# EUROPEAN PATENT APPLICATION

(11) **EP 4 052 741 A1**
(43) Date of publication of application: **07.09.2022**
(21) Application number: 20880690.1
(22) Date of filing: 29.10.2020
(51) Int. Cl.: A61M 5/168, A61M 5/142, A61M 5/14, A61M 5/165, A61M 5/36

(54) **LIQUID MEDICINE PUSH DEVICE AND LIQUID MEDICINE INJECTION DEVICE INCLUDING SAME**

(30) Priority: 01.11.2019 KR 20190138895; 19.03.2020 KR 20200033869; 19.03.2020 KR 20200033875; 19.03.2020 KR 20200033882; 19.03.2020 KR 20200033885
(71) Applicant: Kim, Yong Hyun, Goyang-si, Gyeonggi-do 10483 (KR)
(72) Inventor: Kim, Yong Hyun, Goyang-si, Gyeonggi-do 10483 (KR)
(74) Representative: Santarelli
(86) International application number: PCT/KR2020/014899
(87) International publication number: WO 2021/086046

(57) **Abstract**

A medicinal liquid pushing apparatus according to an embodiment of the present disclosure has a medicinal liquid channel that guides a flow of a medicinal liquid. The medicinal liquid pushing apparatus includes: a reservoir defining an internal space configured to store a medicinal liquid on the medicinal liquid channel; a reservoir pushing member configured to press the reservoir downward; a button member disposed to be spaced apart upward from the reservoir pushing member and configured to be movable downward; and a pressing member disposed between the button member and the reservoir pushing member and configured such that a lower end of the pressing member presses the reservoir pushing member downward while the pressing member is compressively and elastically deformed in an upper-lower direction when an upper end of the pressing member is pushed downward by the button member.

## Description

### TECHNICAL FIELD

The present disclosure relates to a medicinal liquid control apparatus and a medicinal liquid injection apparatus including the same. Here, the medicinal liquid control apparatus relates to a medicinal liquid pushing apparatus, a medicinal liquid storage volume control apparatus, a medicinal liquid supply control apparatus, and a medicinal liquid valve apparatus.

### BACKGROUND

In order to supply a drug to a patient, a medicinal liquid injection apparatus for injecting a medicinal liquid of a liquid state (e.g., an injection solution) to a patient is known. Using the medicinal liquid injection apparatus, the medicinal liquid in a predetermined storage space is introduced into the body of the patient through a passage (e.g., the inner spaces of a tube and an injection needle) connected to a patient.

Among medicinal liquids, there are medicinal liquids (e.g., antibiotics, analgesics, and anticancer drugs) that should be injected into a patient's body in a limited injection dose per hour. In general, such medicinal liquids are injected into the patient's body in a constant dose per hour. However, depending on conditions such as a patient's condition, there is also a need to temporarily increase the supply dose of a medicinal liquid to be injected. Such an injection performed in the state in which the amount of a medicinal liquid is temporarily increased is called a bolus injection.

A medicinal liquid supply control apparatus that enables an injection of a constant dose of a medicinal liquid and the bolus injection is known.

A bolus capacity, which is the maximum amount of a medicinal liquid to be stored after a certain period of time, is set differently depending on a product so that the product is replaced with a product with a different bolus dose if a patient's condition changes.

In this regard, an apparatus that allows a patient to push a button in order to initiate the bolus injection of a medicinal liquid by himself/herself is known. In order to perform the bolus injection according to the prior art, there is an inconvenience in that the user has to keep pressing the injection button until the bolus injection is completed.

Meanwhile, an apparatus for opening/closing a channel through which a medicinal liquid flows is known. An opening/closing apparatus, in which a lever capable of pressing a flexible tube through which a medicinal liquid flows is provided outside the flexible tube, is known. The lever pushes and presses the tube, thereby a channel inside the tube is closed.

### SUMMARY

An embodiment of the present disclosure provides an apparatus capable of determining a start time of the bolus injection of a medicinal liquid or a start time of injecting a predetermined dose of a stored medicinal liquid by a patient himself/herself according to the patient's condition.

In order to perform the bolus injection according to the prior art, there is an inconvenience in that the user has to keep pressing the injection button until the bolus injection is completed. In particular, it is more inconvenient for a patient who is not in a good physical condition to use the injection button for this bolus injection. An embodiment of the present disclosure solves this problem.

When a user releases the pushed state of the injection button in a process of pushing the injection button for the bolus injection according to the prior art, the medicinal liquid is not completely injected and the therapeutic effect may be reduced. An embodiment of the present disclosure solves this problem.

When a user pushes the injection button with an excessive force for the bolus injection according to the prior art, there is a problem in that the inlet dose of a medicinal liquid per hour may increase beyond an appropriate value. An embodiment of the present disclosure solves this problem.

Since a bolus capacity of a conventional product (the storage capacity of a medicinal liquid) is fixed for each product, there is a problem in that a differential application of the medicinal liquid injection dose according to the patient's condition is impossible. An embodiment of the present disclosure solves this problem.

The conventional medicinal liquid supply control apparatus has a continuous line through which a medicinal liquid continuously flows and a bolus line through which the medicinal liquid flows only during the bolus injection. Since the continuous line is unconditionally open until an injection of a prescribed medicinal liquid is completed, there are a problem in that it is difficult to reduce a dose of the medicinal liquid to be injected to the patient and a problem in that it is necessary to also block the bolus line in order to block the continuous line. An embodiment of the present disclosure solves this problem.

Since the conventional medicinal liquid supply control apparatus uses an externally mounted air filter, there is a problem in that it is difficult to discharge air existing or generated between the air filter and a capillary tube in the medicinal liquid supply control apparatus. An embodiment of the present disclosure solves this problem.

An embodiment of the present disclosure provides an apparatus capable of conveniently opening/closing a channel through which a medicinal liquid flows.

An embodiment of the present disclosure provides an apparatus capable of reliably and safely opening/closing a channel through which a medicinal liquid flows.

An aspect of the present disclosure provides embodiments of a medicinal liquid pushing apparatus having a medicinal liquid channel that guides a flow of a medicinal liquid. A medicinal liquid pushing apparatus according to a representative embodiment includes: a reservoir defining an internal space configured to store the medicinal liquid on the medicinal liquid channel; a reservoir pushing member configured to press the reservoir downward; a button member disposed to be spaced apart upward from the reservoir pushing member and configured to be movable downward; and a pressing member disposed between the button member and the reservoir pushing member and configured such that a lower end of the pressing member presses the reservoir pushing member downward while the pressing member is compressively and elastically deformed in an upper-lower direction when an upper end of the pressing member is pushed downward by the button member.

Another aspect of the present disclosure provides embodiments of a medical liquid injection apparatus. A medicinal liquid injection apparatus according to a representative embodiment includes: the medicinal liquid pushing apparatus; a pumping module configured to press the medicinal liquid; and an extension tube configured to allow the medicinal liquid flowing out from the pumping module by being pressed in the pumping module to flow through the extension tube. The medicinal liquid channel of the medicinal liquid pushing apparatus is connected to the extension tube.

Still another aspect of the present disclosure provides embodiments of a medicinal liquid storage volume control apparatus. In a representative embodiment, the medicinal liquid storage volume control apparatus includes a medicinal liquid channel that guides a flow of a medicinal liquid. The medicinal liquid storage volume control apparatus includes: a reservoir that defines an internal space configured to store the medicinal liquid on the medicinal liquid channel and is configured such that a length of the reservoir in an upper-lower direction increases when a volume of the internal space increases; a reservoir pushing member configured to move by being pushed upward by the reservoir when the length of the reservoir in the upper-lower direction increases; and a selector configured to come into contact with the reservoir pushing member to limit a maximum upward movement position of the reservoir pushing member, and configured to be switchable from a first set state in which the maximum upward movement position is set to be a first limit position to a second set state in which the maximum upward movement position is set to be a second limit position lower than the first limit position.

In an embodiment, the selector may be configured to be switchable from the first set state to the second set state when a second contact surface coming into contact with a first contact surface varies, wherein one of the selector and the reservoir pushing member has the first contact surface and the other one of the selector and the reservoir pushing member has the second contact surface.

In an embodiment, the medicinal liquid storage volume control apparatus may be configured such that the first contact surface and the second contact surface are capable of contacting each other in the upper-lower direction.

In an embodiment, the second contact surface in the second set state may be located in a direction facing the first contact surface with reference to the second contact surface in the first set state.

In an embodiment, the one of the selector and the reservoir pushing member may form the first contact surface protruding toward the other one of the selector and the reservoir pushing member and facing the other one.

In an embodiment, one of the selector and the reservoir pushing member may include at least one limiter protruding toward the other one, and the other one of the selector and the reservoir pushing member may include a first level surface and a second level surface with which the limiter is capable of being in contact. The first level surface and the second level surface may have different levels in an upper-lower direction.

In an embodiment, the at least one limiter may include a plurality of limiters arranged to be spaced apart from each other along a circumferential direction of the selector.

In an embodiment, the selector may be configured to be switched from the first set state to the second set state by rotating in the circumferential direction.

In an embodiment, the selector may be configured to be switched from the first set state to the second set state by rotating in a circumferential direction of the selector.

In an embodiment, the selector may include: a selector body extending in the circumferential direction; and at least one limiter protruding downward from the selector body.

In an embodiment, the selector may include: a selector body extending along a circumferential direction; and at least one limiter protruding downward from the selector body.

In an embodiment, the medicinal liquid storage volume control apparatus may further include a case. The case may accommodate the reservoir, the reservoir pushing member, and the selector therein. The selector may include a selector operation portion penetrating the case from the inside to the outside. A selector hole through which the selector operation portion passes may be formed at the case. The selector operation portion may be configured to be disposed at a first set position in the selector hole in the first set state, and may be configured to be disposed at a second set position different from the first set position in the selector hole in the second set state.

In an embodiment, one of the case and the selector may include an engagement portion elastically deformable by the other one. The engagement portion may be configured to be elastically deformed when the selector operation portion moves from the first set position or the second set position in the selector hole to an intermediate position between the first set position and the second set position.

In an embodiment, the selector operation portion may include a head in which a groove is formed in a portion exposed to the outside of the case. A cotter insertable into the groove may be further included.

In an embodiment, the reservoir pushing member may be configured to be capable of pressing the reservoir downward.

In an embodiment, an opening penetrating the selector in an upper-lower direction is formed at the selector. The medicinal liquid storage volume control apparatus may further include a pressing member configured to pass through the opening downward to be capable of pressing the reservoir pushing member downward.

In an embodiment, the medicinal liquid storage volume control apparatus may further include a reservoir support part supporting a lower surface of the reservoir.

In an embodiment, the medicinal liquid channel may include: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel, and is configured to be capable of opening/closing. An internal space of the reservoir may be located on the second channel.

In an embodiment, the medicinal liquid storage volume control apparatus may further include a case surrounding a circumference of the selector. The selector may include: a first selector part extending along an inner circumferential surface of the case and having one end and the other end spaced apart from each other in a circumferential direction; and a ring-shaped second selector part coupled to the inner circumferential surface of the first selector part and extending in the circumferential direction.

A medicinal liquid injection apparatus according to another representative embodiment includes: the medicinal liquid storage volume control apparatus; a pumping module configured to press the medicinal liquid; and an extension tube configured to allow the medicinal liquid flowing out from the pumping module by being pressed in the pumping module to flow through the extension tube. The medicinal liquid channel of the medicinal liquid storage volume control apparatus is connected to the extension tube.

Still another aspect of the present disclosure provides embodiments of a medicinal liquid supply control apparatus. In a representative embodiment, the medicinal liquid supply control apparatus has a medicinal liquid channel that guides a flow of a medicinal liquid. The medicinal liquid channel includes: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel. The medicinal liquid supply control apparatus may include: a reservoir that defines an internal space configured to store the medicinal liquid on the second channel; a first channel valve configured to be capable of switching a section between the branching point and the merging point in the first channel from an open state to a closed state; and a second channel valve configured to open and close the second channel.

In an embodiment, the medicinal liquid supply control apparatus may further include: a reservoir pushing member configured to press the reservoir; and a button member configured to be movable to a predetermined pushing direction so that the reservoir pushing member presses the reservoir and simultaneously the second channel valve opens the second channel, and configured to be movable to a direction opposite to the pushing direction so that the reservoir pushing member releases the pressing on the reservoir and simultaneously the second channel valve closes the second channel.

In an embodiment, the medicinal liquid supply control apparatus may further include: a case accommodating the reservoir, the first channel valve, and the second channel valve therein; a switching member configured to be movable to operate the first channel valve; and a cotter configured to be insertable into the case to lock the button member and configured to be insertable into the case to move the switching member.

In an embodiment, the medicinal liquid supply control apparatus may further include a channel forming part defining a first extension hole constituting a portion of the first channel and a second extension hole constituting a portion of the second channel. The first channel valve may protrude toward one end of the first extension hole. The second channel valve may protrude toward one end of the second extension hole.

In an embodiment, the medicinal liquid supply control apparatus may further include a valve plate at which the first channel valve and the second channel valve are formed. The valve plate may cover a portion of the channel forming part and be configured to be elastically deformed or elastically restored to operate the first channel valve and the second channel valve.

In an embodiment, the valve plate may include: a first pushing portion located at an end in a direction opposite to a protrusion direction of the first channel valve and configured to operate the first channel valve by being pushed or released; and a second pushing portion located at an end in a direction opposite to a protrusion direction of the second channel valve and configured to operate the second channel valve by being pushed or released.

In an embodiment, the medicinal liquid supply control apparatus may further include a switching member configured to be movable to operate the first channel valve independently from the second channel valve.

In an embodiment, the medicinal liquid supply control apparatus may further include: a channel forming part defining a first extension hole constituting a portion of the first channel; a pushing portion located at a lower end of the first channel valve; and a switching member defining a groove into which a lower end of the pushing portion is insertable. The first channel valve may protrude upward toward one end of the first extension hole. The first channel valve may be configured to open the one end of the first extension hole in a released state in which the lower end of the pushing portion is inserted into the groove of the switching member. The first channel valve may be configured to block the one end of the first extension hole in a locked state in which the lower end of the pushing portion is pressed upward by an upper surface of the switching member.

In an embodiment, the reservoir may be configured such that a length of the reservoir in a predetermined direction increases when a volume of the internal space increases. The medicinal liquid supply control apparatus may include: a reservoir pushing member configured to move by being pushed in the predetermined direction by the reservoir when the length of the reservoir in the predetermined direction increases; and a selector configured to come into contact with the reservoir pushing member to limit a maximum movement position of the reservoir pushing member in the predetermined direction, and configured to be capable of performing an operation of selecting one of a plurality of set states in which the maximum movement positions in the predetermined direction are set to be different from each other.

In an embodiment, an air passage branched from the medicinal liquid channel and connected to the outside is formed at the medicinal liquid supply control apparatus. The medicinal liquid supply control apparatus may further include: a hydrophobic air passing filter disposed at a boundary between the air passage and the medicinal liquid channel; a filter cap on which the air passing filter is disposed and which defines the air passage; and a filter spacer inserted into the filter cap to space the air passing filter from an inner surface of the filter cap that defines the air passage.

A medicinal liquid injection apparatus according to another representative embodiment includes: the medicinal liquid supply control apparatus; a pumping module configured to press the medicinal liquid; and an extension tube configured to allow the medicinal liquid flowing out from the pumping module by being pressed in the pumping module to flow through the extension tube. The medicinal liquid channel of the medicinal liquid supply control apparatus is connected to the extension tube.

Still another aspect of the present disclosure provides embodiments of a medicinal liquid valve apparatus. In a representative embodiment, the medicinal liquid valve apparatus has a medicinal liquid channel that guides a flow of a medicinal liquid. The medicinal liquid valve apparatus includes: a channel forming part defining an extension hole constituting a portion of the medicinal liquid channel, one end of the extension hole being located on a lower surface of the channel forming part; and a valve plate including a channel valve protruding upward toward the one end of the extension hole and a pushing portion located at a lower end of the channel valve. The valve plate covers at least a portion of the lower surface of the channel forming part. In a locked state in which the pushing portion is pressed upward, the valve plate is elastically deformed so that the channel valve blocks the one end of the extension hole. In a released state in which the upward pressing of the pushing portion is released, the valve plate is elastically restored so that the channel valve opens the one end of the extension hole.

In an embodiment, a guide channel partitioned by the channel forming part and the valve plate may be formed between the channel forming part and the valve plate. In the released state, the extension hole and the guide channel may be connected through the one end of the extension hole to constitute a portion of the medicinal liquid channel.

In an embodiment, a gap may be formed along a circumference of the channel valve between the channel forming part and the valve plate. In the released state, the extension hole and the gap may be connected through the one end of the extension hole to constitute a portion of the medicinal liquid channel.

In an embodiment, the pushing portion may protrude downward.

In an embodiment, the medicinal liquid valve apparatus may further include a valve support part supporting a lower surface of the valve plate. A pushing portion penetration hole through which the pushing portion passes downward may be formed in the valve support part. A lower end of the pushing portion may be configured to be capable of pressing upward.

In an embodiment, the valve plate may further include an inclined portion extending obliquely upward from a circumference of the pushing portion in the released state.

In an embodiment, the valve plate may be made of a material that is more flexible than that of the channel forming part.

In an embodiment, the medicinal liquid channel may include: a first channel that guides the medicinal liquid to flow from an inlet to an outlet; and a second channel that guides the medicinal liquid to split at a branching point of the first channel, guides the split medicinal liquid to merge at a merging point located downstream of the branching point of the first channel. The medicinal liquid valve apparatus may further include a reservoir that defines an internal space configured to store the medicinal liquid on the second channel. The extension hole may constitute a portion of the first channel or a portion of the second channel in a section between the branching point and the merging point in the medicinal liquid channel.

In an embodiment, the medicinal liquid valve apparatus may further include a lock bar configured to be movable in an upper-lower direction. The lock bar may be configured to move upward to press the pushing portion upward when the released state is switched to the locked state.

In an embodiment, the medicinal liquid valve apparatus may further include a lock elastic member supporting the lock bar and configured to be elastically deformed when the lock bar moves downward.

In an embodiment, the medicinal liquid valve apparatus may further include a button member configured to be movable in an upper-lower direction. The button member may be configured to move downward to press the lock bar downward when the locked state is switched to the released state.

In an embodiment, the medicinal liquid valve apparatus may further include a switching member defining a groove into which a lower end of the pushing portion is inserted in the released state. The switching member may be configured such that the lower end of the pushing portion is pressed upward by an upper surface of the switching member in the locked state.

In an embodiment, the medicinal liquid valve apparatus may further include a valve support part supporting a lower surface of the valve plate. The switching member may be configured to be movable in a direction transverse to an upper-lower direction on the valve support part. The groove may be formed in the upper surface of the switching member.

In an embodiment, a switching member arrangement hole, into which the switching member is inserted, is formed on a side surface of the valve support part. The medicinal liquid valve apparatus may further include a cotter configured to be capable of pressing the switching member in the direction transverse to the upper-lower direction.

In an embodiment, one of the valve support part and the switching member includes a position setting protrusion protruding toward the other one, and a positioning groove into which the position setting protrusion is insertable is formed at the other one of the valve support part and the switching member. The position setting protrusion may be configured to be engaged with the positioning groove in the locked state.

A medicinal liquid injection apparatus according to still another representative embodiment includes: the medicinal liquid valve apparatus; a pumping module configured to press the medicinal liquid; and an extension tube configured to allow the medicinal liquid flowing out from the pumping module by being pressed in the pumping module to flow through the extension tube. The medicinal liquid channel of the medicinal liquid valve apparatus is connected to the extension tube.

According to embodiment of the present disclosure, an apparatus capable of determining a start time of the bolus injection of a medicinal liquid or a start time of injecting a predetermined dose of a stored medicinal liquid by a patient himself/herself according to the patient's condition is provided.

According to an embodiment of the present disclosure, it is not necessary for a patient to keep pressing the button for the bolus injection. Thus, convenient use is possible, and the therapeutic effect can be increased by allowing a medicinal liquid to be completely injected.

According to an embodiment of the present disclosure, the possibility of causing unnecessary pain to a patient can be significantly reduced by preventing a speed of the bolus injection from being excessively fast.

According to an embodiment of the present disclosure, a differential application of a medicinal liquid injection dose depending on a patient's condition can be conveniently performed without replacing the product.

According to an embodiment of the present disclosure, it is possible to block only the continuous channel among the continuous channel and the bolus channel of the medicinal liquid supply control apparatus. Therefore, it is very convenient to control a medicinal liquid injection dose to the patient.

According to an embodiment of the present disclosure, it is possible to easily discharge air existing or generated in the medicinal liquid supply control apparatus.

According to embodiments of the present disclosure, it is possible to conveniently, reliably, and safely open/close the channel through which a medicinal liquid flows.

According to the embodiments of the present disclosure, it is possible to prevent leakage of a medicinal liquid to the outside of the channel while making it possible to block the flow of the medicinal liquid inside the channel.

### BRIEF DESCRIPTION OF THE DRAWINGS

FIG. 1 is a conceptual diagram illustrating an entire system of a medicinal liquid injection apparatus according to an embodiment of the present disclosure.
FIG. 2 is a perspective view of a medicinal liquid control apparatus according to an embodiment of the present disclosure, illustrating a figure in which a user operates a button member in a state in which a cotter is removed.
FIG. 3 is a perspective view of the medicinal liquid control apparatus of FIG. 2, illustrating manners in which cotters can be coupled to the medicinal liquid control apparatus.
FIG. 4 is a perspective view illustrating a state in which a first case part and a second case part are removed from the medicinal liquid control apparatus of FIG. 3.
FIG. 5 is a perspective view illustrating a state in which a guide part is additionally removed from the medicinal liquid control apparatus of FIG. 4.
FIG. 6 is an exploded perspective view of the medicinal liquid control apparatus of FIG. 3.
FIG. 7 is an exploded perspective view of the medicinal liquid control apparatus of FIG. 6, viewed from another angle.
FIG. 8 is an exploded perspective view illustrating only some of the components of FIG. 6, in which unlike FIG. 6, some components are illustrated in a state of being assembled with each other.
FIG. 9 is an exploded perspective view viewed from another angle of FIG. 8, in which an enlarged view (E) of a portion is illustrated.
FIG. 10 is a perspective view illustrating a cross section of the medicinal liquid control apparatus taken along line S1-S1' in FIG. 9.
FIG. 11 is a cross-sectional view of the medicinal liquid control apparatus taken along line S2-S2' in FIG. 9.
FIG. 12 is a cross-sectional view of the medicinal liquid control apparatus taken along line S3-S3' in FIG. 9.
FIG. 13 is a cross-sectional view of the medicinal liquid control apparatus taken along line S4-S4' in FIG. 9.
FIG. 14 is an exploded perspective view illustrating the button member, a reservoir pushing member, a pressing member, a support slider, and a slider spring in FIG. 6.
FIG. 15 is an exploded perspective view illustrating the button member, the reservoir pushing member, the pressing member, the support slider, and the slider spring in FIG. 6.
FIG. 16 is a longitudinal cross-sectional view illustrating an operation mechanism of the button member, the reservoir pushing member, the pressing member, the support slider, and the slider spring.
FIG. 17 is a longitudinal cross-sectional view illustrating an operation mechanism of the button member, the reservoir pushing member, the pressing member, the support slider, and the slider spring.
FIG. 18 is a longitudinal cross-sectional view illustrating an operation mechanism of the button member, the reservoir pushing member, the pressing member, the support slider, and the slider spring.
FIG. 19 is a longitudinal cross-sectional view illustrating an operation mechanism of the button member, the reservoir pushing member, the pressing member, the support slider, and the slider spring.
FIG. 20 is a longitudinal cross-sectional view illustrating an operation mechanism of the button member, the reservoir pushing member, the pressing member, the support slider, and the slider spring.
FIG. 21 is a cross-sectional view of the medicinal liquid control apparatus taken along line S6-S6' in FIG. 17.
FIG. 22 is a cross-sectional view of the medicinal liquid control apparatus taken along line S7-S7' in FIG. 19.
FIG. 23 is a partial longitudinal cross-sectional conceptual view of a medicinal liquid control apparatus including support sliders 1600' and slider springs 1700' according to another embodiment, illustrating a state before the button member is lowered.
FIG. 24 is a partial longitudinal cross-sectional conceptual view of the medicinal liquid control apparatus including the support sliders 1600' and the slider springs 1700' according to another embodiment, illustrating a state in which the button member is lowered.
FIG. 25 is a cross-sectional conceptual view taken along line S8-S8' in FIG. 23.
FIG. 26 is a cross-sectional conceptual view taken along line S9-S9' in FIG. 24.
FIG. 27 is an exploded perspective view of the reservoir pushing member, a selector, and the cotter of FIG. 6.
FIG. 28 is an exploded perspective view of the reservoir pushing member, the selector, and the cotter of FIG. 6.
FIG. 29 is a perspective view illustrating a state in which the case is disassembled in the medicinal liquid control apparatus of FIG. 3.
FIG. 30 is a view illustrating a state in which the selector is operated by using the cotter in the medicinal liquid control apparatus of FIG. 3.
FIG. 31 is a view illustrating a state in which the selector is operated by using the cotter in the medicinal liquid control apparatus of FIG. 3.
FIG. 32 is a view illustrating a state in which the selector is operated by using the cotter in the medicinal liquid control apparatus of FIG. 3.
FIG. 33 is a perspective view illustrating a state in which the case is transparently processed in the medicinal liquid control apparatus of FIG. 30.
FIG. 34 is a perspective view illustrating a state in which the case is transparently processed in the medicinal liquid control apparatus of FIG. 32.
FIG. 35 is a cross-sectional view of the medicinal liquid control apparatus taken along line S10-S10' in FIG. 30.
FIG. 36 is a cross-sectional view of the medicinal liquid control apparatus taken along line S11-S11' in FIG. 31.
FIG. 37 is a view illustrating some components including a lock bar, a lock elastic member, a switching member, and the like in a disassembled state.
FIG. 38 is a view illustrating some components including a lock bar, a lock elastic member, a switching member, and the like in a disassembled state.
FIG. 39 is a cross-sectional view of some components of the medicinal liquid control apparatus taken along line S12-S12' in FIG. 37, illustrating a state in which a channel P2 is blocked by a valve 318.
FIG. 40 is a cross-sectional view illustrating a state in which the button member is lowered by a partial section from FIG. 39 and the button member and the lock bar are in contact with each other.
FIG. 41 is a cross-sectional view illustrating a state in which the button member is further lowered from FIG. 40 to move the lock bar so that the channel P2 is opened by the valve 318.
FIG. 42 is a perspective view illustrating a state in which the switching member is separated from the medicinal liquid control apparatus of FIG. 3.
FIG. 43 is a partial cross-sectional view of the medicinal liquid control apparatus taken along line S13-S13' of FIG. 42, illustrating a state in which a channel P1 is opened by a valve 316.
FIG. 44 is a partial cross-sectional view of the medicinal liquid control apparatus taken along line S13-S13' in FIG. 42, illustrating a state in which the channel P1 is blocked by the valve 316 by the movement of the switching member from FIG. 43.
FIG. 45 is an exploded perspective view of a filter body, a filter cap, a first air-pass filter, a second air passing filter, a boundary filter, and a filter spacer of FIGS. 6 and 7.
FIG. 46 is an exploded perspective view illustrating a state in which the filter cap and the filter spacer of FIG. 45 are separated from each other.
FIG. 47 is a cross-sectional view of an assembly of the components of FIG. 45 taken along line S12-S12' in FIG. 43.

### DETAILED DESCRIPTION

Embodiments of the present disclosure are illustrated for the purpose of explaining the technical idea of the present disclosure. The scope of the rights according to the present disclosure is not limited to the embodiments presented below or the detailed descriptions of such embodiments.

All technical and scientific terms used in the present disclosure have the meaning generally understood by those of ordinary skill in the art to which the present disclosure pertains, unless otherwise defined. All terms used in the present disclosure are chosen for the purpose of more clearly describing the present disclosure and are not chosen to limit the scope of rights according to the present disclosure.

As used in the present disclosure, expressions such as "comprising," "including," "having," and the like are to be understood as open-ended terms having the possibility of encompassing other embodiments, unless otherwise mentioned in the phrase or sentence containing such expressions.

The singular form described in the present disclosure may include a plural meaning, unless otherwise mentioned. This applies equally to the singular form recited in the claims.

The terms "first," "second," and the like used in the present disclosure are used to distinguish a plurality of components from one another and do not limit the order or importance of the corresponding components.

In the present disclosure, where it is mentioned in the present disclosure that one element is "connected" or "coupled" to another element, it is to be understood that said one element may be directly connected or coupled to another element or may be connected or coupled to said another element via a new additional element.

Direction indicating words such as "upward," "upper," and the like used in the present disclosure mean a direction of arrow U in the accompanying drawings, and direction indicating words such as "downward," "lower," and the like mean a direction of arrow D in the accompanying drawings, but these are merely references for explaining the present disclosure such that the present disclosure can be clearly understood, and depending on what is the reference, the direction indicating words may be interpreted accordingly.

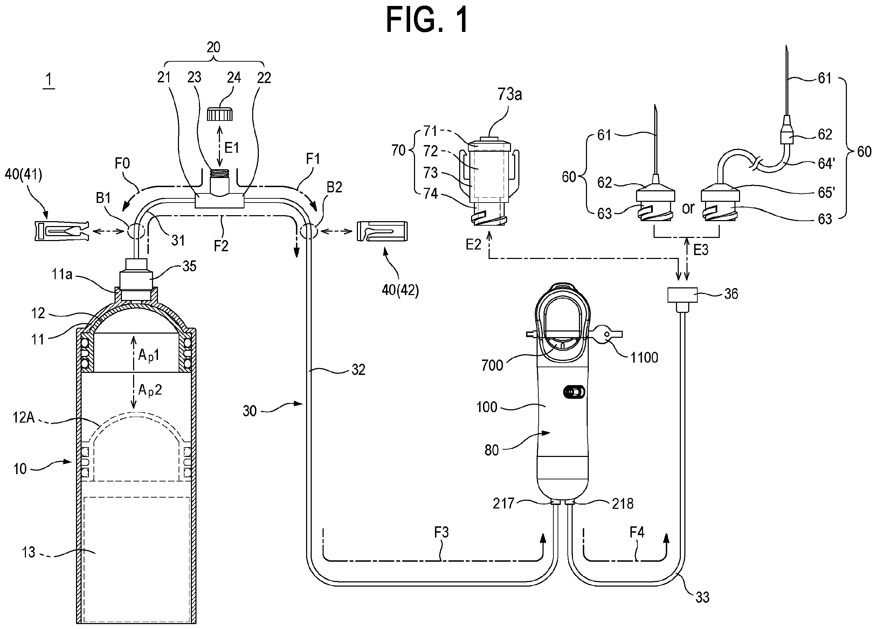

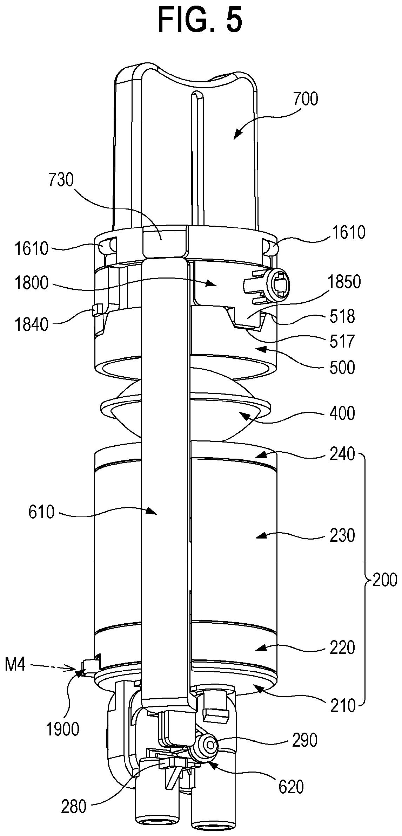

"Upstream" and "downstream" used in the present disclosure are defined based on a direction in which a medicinal liquid flows when a pumping module 10 presses the medicinal liquid. Specifically, directions of arrows F2 and F3 of FIG. 1 and arrow F4 of FIG. 5 are defined as a downstream direction, and a direction opposite to the downstream direction is defined as an upstream direction.

A "medicinal liquid" used in the present disclosure should be understood to include not only a liquid containing a therapeutic substance, but also a liquid that assists the therapeutic substance or can be used together with the therapeutic substance, and a liquid that can be injected into a patient. A priming liquid, which will be described later, is one of these medicinal liquids.

Hereinafter, embodiments of the present disclosure will be described with reference to the accompanying drawings. In the accompanying drawings, identical or corresponding components may be indicated by identical reference numerals. In the following description of embodiments, repeated descriptions of the identical or relevant components will be omitted. However, even if a description of a component is omitted, such a component is not intended to be excluded in an embodiment.

FIG. 1 is a conceptual view illustrating the entire system of a medicinal liquid injection apparatus 1 according to an embodiment of the present disclosure. The process of injecting a medicinal liquid into a patient using the medicinal liquid injection apparatus 1 according to embodiments of the present disclosure includes a priming step and a medicinal liquid injection step, which proceed sequentially.

Referring to FIG. 1, in the priming step, a priming liquid flows along an extension tube 30. The priming liquid flowing along the extension tube 30 is introduced into a medicinal liquid control apparatus 80. The medicinal liquid injection apparatus 1 may include an end cap 70 detachably connected to the downstream side of the medicinal liquid control apparatus 80. Air inside the extension tube 30 may be discharged to the outside through the end cap 70.

Accordingly, the interiors of the extension tube 30 and the medicinal liquid control apparatus 80 are filled with the priming liquid. The priming liquid may be a liquid containing a therapeutic substance or a liquid that can be injected into a patient, such as a saline solution.

The end cap 70 is configured such that the air passing through the medicinal liquid control apparatus 80 and the priming liquid are introduced into the end cap 70. The end cap 70 may be configured to allow air to flow out to the outside, but to prevent the priming liquid from flowing out to the outside.

The end cap 70 includes a vent filter 71 that blocks the passage of the priming liquid therethrough but allows the passage of gas therethrough. The vent filter 71 includes a hydrophobic filter. The end cap 70 may include a sponge 72 disposed upstream of the vent filter 71. The end cap 70 includes an end cap casing 73 accommodating the vent filter 71 therein. The end cap casing 73 accommodates the sponge 72 therein. The end cap casing 73 provides a vent hole 73a through which the gas passes. The end cap 70 includes an end cap coupling portion 74 configured to be coupled with a downstream connection portion 36 constituting the downstream end of the extension tube 30 of the medicinal liquid injection apparatus 1. Arrow E2 in FIG. 1 illustrates the coupling and decoupling direction of the end cap coupling portion 74 with respect to the downstream connection portion 36.

When the end cap 70 is filled with the priming liquid, the end cap 70 may be separated from the medicinal liquid control apparatus 80, and the medicinal liquid control apparatus 80 and a patient connection unit 60 or 60' may be connected to each other.

The patient connection unit 60 or 60' may include an injection needle 61, a catheter, or the like. The patient connection unit 60 or 60' includes a component that is introduced into a patient's body, such as the injection needle 61.

The patient connection unit 60 or 60' may include "an introduction component including a component to be introduced into the patient's body, such as the injection needle 61" and "a remaining component." The introduction component and the remaining component may be detachably coupled to each other. In this case, in the state in which the introduction component is connected to the patient but is separated from the remaining component, the user may couple the remaining component to the medicinal liquid control apparatus 80 and may then couple the introduction component and the remaining component to each other. In this case, the liquid passing through the medicinal liquid control apparatus 80 may be introduced into the patient's body after sequentially passing through the remaining component and the introduction component.

The patient connection unit 60 or 60' includes an injection support portion 62 that supports the injection needle 61. The patient connection unit 60 or 60' includes a unit coupling portion 63 configured to be coupled with the downstream connection portion 36 of the medicinal liquid injection apparatus 1. Arrow E3 in FIG. 1 illustrates the direction of coupling and decoupling the unit coupling portion 63 with respect to the downstream connection portion 36.

As an example, the patient connection unit 60 may be configured such that the injection needle 61, the injection support portion 62, and the unit coupling portion 63 are sequentially connected.

As another example, the patient connection unit 60' further includes a patient connection tube fixing portion 65' connected to the downstream side of the unit coupling portion 63. The patient connection unit 60' further includes a patient connection tube 64' that interconnects the patient connection tube fixing portion 65' and the injection support portion 62. The patient connection tube 64' may be formed of a flexible material. The patient connection unit 60' may be configured by sequentially connecting the injection needle 61, the injection support portion 62, the patient connection tube 64', the patient connection tube fixing portion 65', and the unit coupling portion 63.

In the medicinal liquid injection step, the medicinal liquid is introduced into the patient's body in response to the pressing in the pumping module 10. The medicinal liquid in the medicinal liquid injection step is a liquid containing a therapeutic substance. When the priming liquid is not a liquid containing a therapeutic substance but a saline solution, the priming liquid first flows into the patient's body, and the liquid containing the therapeutic substance that flows following the priming liquid may be introduced into the patient's body.

The pumping module 10 is configured to press a medicinal liquid. The pumping module 10 includes a chamber 11 configured to accommodate a medicinal liquid. The chamber 11 defines an internal space with a pressing unit 12. A medicinal liquid may be stored in the internal space. In another embodiment, a saline solution and the like may be temporarily stored in the internal space. In the chamber 11, a discharge port portion 11a through which the liquid inside the chamber 11 is discharged is provided.

The pumping module 10 may include a pressing operation portion 13 that provides power to move the pressing unit 12 in a pressing direction Ap1. As an example, the pressing operation portion 13 may be configured to press the liquid in the chamber 11 by using a volumetric expansion by gas activation. As another example, the pressing operation portion 13 may provide a portion that can be held by the user, and may move the pressing unit 12 in the pressing direction Ap1 with the force of the user.

Although not illustrated, as another example, the pressing unit 12 may be configured to press the liquid by using the elastic force of an elastic body such as a balloon. In this case, the pressing unit 12 may be configured to press the liquid in the balloon.

A medicinal liquid injection valve 20 is configured to be capable of filling the chamber 11 with the liquid. The liquid may be introduced into the extension tube 30 or the chamber 11 through the medicinal liquid injection valve 20 from the outside. The medicinal liquid injection valve 20 is connected to the extension tube 30, but in another embodiment (not illustrated), the medicinal liquid injection valve 20 may be connected to the chamber 11.

The medicinal liquid injection valve 20 includes a first extension portion 21 connected to the downstream end of a first connection portion 31 of the extension tube 30, and a second extension portion 22 connected to the upstream end of a second connection portion 32 of the extension tube 30. The medicinal liquid injection valve 20 includes an inlet portion 23 configured to allow liquid to be introduced from the outside therethrough and an inlet port opening/closing part 24 configured to be detachably coupled to the inlet portion 23. Arrow E1 in FIG. 1 indicates the direction of coupling/decoupling the inlet port opening/closing part 24 with respect to the inlet portion 23.

The extension tube 30 is configured to guide the flow of the priming liquid. The extension tube 30 may guide the movement of the medicinal liquid from the pumping module 10 to the medicinal liquid control apparatus 80.

The extension tube 30 is configured to allow the medicinal liquid flowing out from the pumping module 10 according to the pressing in the pumping module 10 to flow therethrough. The upstream end of the extension tube 30 is connected to the pumping module 10. The extension tube 30 includes an upstream connection portion 35 connected to the discharge port portion 11a of the pumping module 10. The extension tube 30 includes the downstream connection portion 36 connected to the end cap 70 or the patient connection unit 60 or 60'. The liquid passing through the upstream sections 31 and 32 of the extension tube 30 flows into the medicinal liquid control apparatus 80 through an inlet connection portion 217 of the medicinal liquid control apparatus 80, and flows into a downstream section 33 of the extension tube 30 through an outlet connection portion 218 of the medicinal liquid control apparatus 80.

The extension tube 30 includes the first connection portion 31 connecting the upstream connection portion 35 and the first extension portion 21 of the medicinal liquid injection valve 20. The extension tube 30 includes the second connection portion 32 interconnecting the second extension portion 22 of the medicinal liquid injection valve 20 and the inlet connection portion 217 of the medicinal liquid control apparatus 80. The extension tube 30 includes a third connection portion 33 interconnecting the outlet connection portion 218 of the medicinal liquid control apparatus 80 and the downstream connection portion 36.

The medicinal liquid injection apparatus 1 may include at least one connection tube opening/closing module 40. The connection tube opening/closing module 40 may press the outside of the extension tube 30 to block the flow of the liquid at a point in the extension tube 30. The at least one connection tube opening/closing module 40 may include a first connection tube opening/closing module 41 capable of changing whether to open or close a point B1 of the first connection portion 31, and a second connection tube opening/closing module 42 capable of changing whether to open or close a point B2 of the second connection portion 32. For example, the connection tube opening/closing module 40 may be configured in a clamp shape.

In another embodiment (not illustrated), the medicinal liquid injection apparatus 1 may include a filter module (not illustrated) outside the medicinal liquid control apparatus 80 disposed on the extension tube 30, but in the present embodiment, the medicinal liquid control apparatus 80 includes a filter to be described later. The filter may include a particle filter for filtering out impurities and/or an air filter for filtering out air bubbles.

A priming step and a medical liquid injection step according to an embodiment will be described as follows. In the priming step according to the embodiment, a saline solution, which is not a medicinal liquid, is used as the priming liquid. In the priming step according to the embodiment, the inlet port opening/closing part 24 is separated from the inlet portion 23, the first connection portion 31 is blocked by the first connection tube opening/closing module 41 *(see* B1), and the remaining portion of the extension tube 30 except for the first connection portion 31 is opened. Referring to arrows F1, F3, and F4, the priming liquid such as a saline solution flows sequentially through the inlet portion 23, the second extension portion 22, the second connection portion 32, the medicinal liquid control apparatus 80, and the third connection portion 33, so that the interior of the extension tube 30 and the interior of the medicinal liquid control apparatus 80 are filled with the priming liquid.

After the priming step according to the embodiment, the medicinal liquid injection step according to the embodiment proceeds. In the medicinal liquid injection step according to the embodiment, the inlet port opening/closing part 24 is separated from the inlet portion 23, the second connection portion 32 is blocked with the second connection tube opening/closing module 42 *(see* B2), and the first connection tube opening/closing module 41 is separated from the first connection portion 31 to open the first connection portion 31. Referring to arrow F0, as the medicinal liquid flows into the chamber 11 through the medicinal liquid injection valve 20 and the first connection portion 31, the pressing unit 12 moves in the direction Ap2. Thereafter, the inlet port opening/closing part 24 is coupled to the inlet portion 23, and the second connection tube opening/closing module 42 is separated from the second connection portion 32 to open the extension tube 30. Referring to arrows F2, F3, and F4, thereafter, by moving the pressing unit 12 in the pressing direction Ap1, the medicinal liquid may pass sequentially through the upstream sections 31 and 32 of the extension tube, the medicinal liquid control apparatus 80, and the downstream section 33 of the extension tube.

A priming step and a medical liquid injection step according to another embodiment will be described as follows. In the priming step according to the other embodiment, a medicinal liquid is used as the priming liquid. In the priming step according to the other embodiment, the chamber 11 is filled with the medicinal liquid, the end cap 70 is connected with the downstream connection portion 36, the inlet portion 23 is blocked with the inlet port opening/closing part 24, and the connection tube opening/closing module 40 is separated from the extension tube 30 to make the extension tube 30 open. Referring to arrows F2, F3, and F4, thereafter, by moving the pressing unit 12 in the pressing direction Ap1, the medicinal liquid, which is the priming liquid, flows sequentially through the upstream sections 31 and 32 of the extension tube, the medicinal liquid control apparatus 80, and the downstream section 33 of the extension tube, so that the interior of the extension tube 30 and the interior of the medicinal liquid control apparatus 80 are filled with the priming liquid.

After the priming step according to the other embodiment, a medicinal liquid injection step according to the other embodiment is performed. In the medicinal liquid injection step according to the other embodiment, referring to arrows F2, F3, and F4, the pressing unit 12 is further moved in the pressing direction Ap1, so that the medicinal liquid can pass sequentially through the upstream sections 31 and 32 of the extension tube, the medicinal liquid control apparatus 80, and the downstream section 33 of the extension tube.

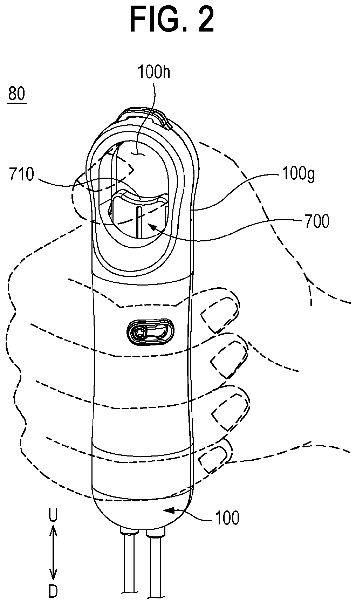

FIG. 2 is a perspective view of a medicinal liquid control apparatus 80 according to an embodiment of the present disclosure, illustrating the figure in which a user operates a button member 700 in a state in which a cotter 1100 is removed.

Referring to FIGS. 1 and 2, the medicinal liquid injection apparatus 1 includes the medicinal liquid control apparatus 80 including a medicinal liquid channel connected to the extension tube 30. The medicinal liquid control apparatus 80 includes a medicinal liquid channel that guides the flow of a medicinal liquid. The medicinal liquid control apparatus 80 may include the inlet connection portion 217 connected to a portion 32 of the extension tube of an upstream side and the outlet connection portion 218 connected to a portion 33 of the extension tube of a downstream side. The medicinal liquid may flow into the medicinal liquid control apparatus 80 through an inlet Qi of the inlet connection portion 217 and flow out to the outside of the medicinal liquid control apparatus 80 through an outlet Qo of the outlet connection portion 218.

The medicinal liquid channel includes a first channel P1 that guides the medicinal liquid to flow from the inlet Qi to the outlet Qo. In the state in which the channel is not closed by the connection tube opening/closing module 40 or a first channel valve 316 to be described later, the first channel P1 may guide the medicinal liquid to continuously flow from the inlet Qi to the outlet Qo.

The medicinal liquid channel may include a second channel P2 that guides the medicinal liquid to split at a branching point Qd, which will be described later, of the first channel P1. The second channel P2 may guide the split flows of the medicinal liquid to be merged at a merging point Qu, which will be described later, located downstream of the branching point Qd of the first channel P1. The second channel P2 may be configured to be capable of opening/closing.

According to an embodiment, the medicinal liquid control apparatus 80 may be variously modified and used. For example, the medicinal liquid control apparatus 80 may be a medicinal liquid pushing apparatus, a medicinal liquid storage volume control apparatus, a medicinal liquid supply control apparatus, or a medicinal liquid valve apparatus.

In a first embodiment, the medicinal liquid control apparatus 80 may be a medicinal liquid pushing apparatus 80 in which a user presses the stored medicinal liquid to inject the stored medicinal liquid into a patient. In the first embodiment, the medicinal liquid pushing apparatus 80 may be a medicinal liquid supply control apparatus operated to increase the dose of a medicinal liquid supplied to a patient by the patient himself/herself, but in another embodiment (not illustrated), the medicinal liquid pushing apparatus 80 may be an apparatus operated to initiate the injection of the medicinal liquid by the patient himself/herself. In the first embodiment, the medicinal liquid pushing apparatus 80 may include a first channel P1 and a second channel P2, and may be configured to be capable of pressing a reservoir 400 having an internal space 400s located on the second channel P2. However, the medicinal liquid channel of the medicinal liquid pushing apparatus 80 may be configured in various manners and is not limited to the present embodiment. For example, the medicinal liquid channel may be a single channel that is not branched (i.e., a channel including only the first channel without the second channel), and the medicinal liquid pushing apparatus 80 may be configured to be capable of pressing the reservoir having an internal space located on the single channel.

In a second embodiment, the medicinal liquid control apparatus 80 may be a medicinal liquid storage volume control apparatus 80 configured to store a medicinal liquid over time so that the medicinal liquid can be injected into a patient, and to be capable of changing the maximum medicinal liquid storage volume. In the second embodiment, the medicinal liquid storage volume control apparatus 80 is an apparatus operated to increase the dose of the medicinal liquid supplied to a patient by the patient himself/herself, but in another embodiment (not illustrated), the medicinal liquid storage volume control apparatus 80 may be an apparatus that is operated to initiate the injection of the medicinal liquid by the patient himself/herself. In the second embodiment, the medicinal liquid storage volume control apparatus 80 may include a first channel P1 and a second channel P2, and may be configured to change the maximum medicinal liquid storage volume of the reservoir 400 having the internal space 400s located on the second channel P2. However, the medicinal liquid channels of the medicinal liquid storage volume control apparatus 80 may be configured in various manners and are not limited to the present embodiment. For example, the medicinal liquid channel may be a single channel that is not branched (i.e., a channel including only the first channel without the second channel), and the medicinal liquid storage volume control apparatus 80 may be configured to be capable of changing the maximum medicinal liquid storage volume of the reservoir having an internal space located on the single channel.

In a third embodiment, the medicinal liquid control apparatus 80 may be a medicinal liquid supply control apparatus 80 that is operated to increase the dose of the medicinal liquid to be supplied to a patient by the patient himself/herself. In the third embodiment, the medicinal liquid supply control apparatus 80 is an apparatus that includes a first channel P1 and a second channel P2 and is configured to enable a bolus injection of the medicinal liquid stored in the reservoir 400 having the internal space 400s located on the second channel P2.

In a fourth embodiment, the medicinal liquid control apparatus 80 may be a medicinal liquid valve apparatus 80 that irreversibly switches a partial section of the medicinal liquid channel from an open state to a closed state, or a medicinal liquid valve apparatus 80 that reversibly opens/closes a partial section of the medicinal liquid channel. In the fourth embodiment, the medicinal liquid valve apparatus 80 is an apparatus operated to increase the dose of a medicinal liquid supplied to a patient by the patient himself/herself, but in another embodiment (not illustrated), the medicinal liquid valve apparatus 80 may be an apparatus that performs a function of simply opening/closing the medicinal liquid channel. In the fourth embodiment, the medicinal liquid valve apparatus 80 may include a first channel P1 and a second channel P2, and may be configured to performs a function of opening or closing the first channel P1 and/or the second channel P2. However, the medicinal liquid channel of the medicinal liquid valve apparatus 80 may be configured in various manners and is not limited to the present embodiment. For example, the medicinal liquid channel may be a single channel that is not branched (i.e., a channel including only the first channel without the second channel), and the medicinal liquid valve apparatus 80 may include a channel valve on the single channel.

Hereinafter, respective configurations of the medicinal liquid control apparatus 80 will be described in more detail, but these are merely configurations according to embodiments of the medicinal liquid control apparatus 80.

Referring to FIG. 2, the medicinal liquid control apparatus 80 may include a case 100 that forms an external appearance. The medicinal liquid control apparatus 80 may include the button member 700 provided to be operable by a user. The medicinal liquid control apparatus 80 may include a cotter 1100. An operation part 710, which is a part of the button member 700, may be exposed to the outside of the case 100. A user (e.g., a patient) may push the operation part 710 to adjust the supply dose of the medicinal liquid. The cotter 1100 may be inserted into the case 100 to prevent the button member 700 from being operated before user's operation. The cotter 1100 is inserted into the case 100 in the state of pushing the operation part 710 downward. When the cotter 1100 is separated from the case 100, the button member 700 moves upward by a pressing member 1000 or an elastic member SP, which will be described later, to be in the state in which the user is capable of pushing the button member 700 downward.

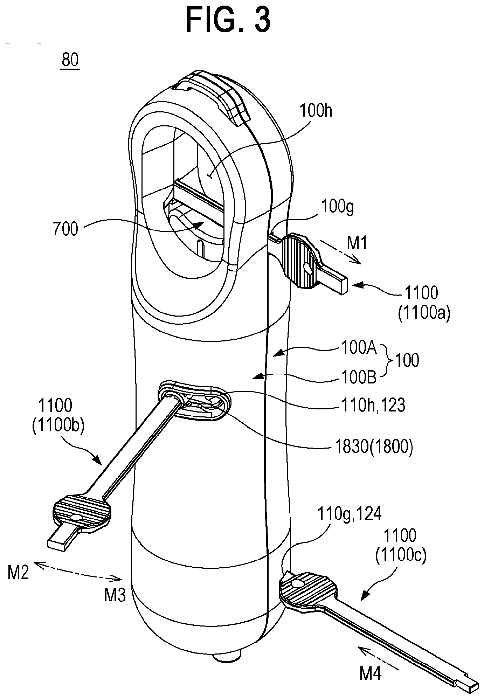

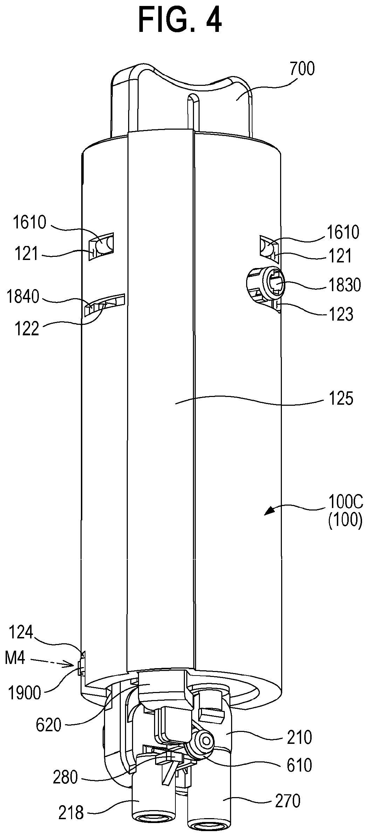

FIG. 3 is a perspective view of the medicinal liquid control apparatus 80 of FIG. 2, illustrating manners in which cotters 1100 can be coupled to the medicinal liquid control apparatus 80. FIG. 4 is a perspective view illustrating the state in which a first case part 100A and a second case part 100B are removed from the medicinal liquid control apparatus 80 of FIG. 3. FIG. 5 is a perspective view illustrating the state in which a guide part 100C is additionally removed from the medicinal liquid control apparatus 80 of FIG. 4.

Referring to FIGS. 3 to 5, the medicinal liquid control apparatus 80 may include a plurality of cotters 1100 provided to suit each purpose, but one cotter 1100 for a medical worker of a medical team or the like may be provided and the one cotter 1100 coupled to the medicinal liquid control apparatus 80 at one of three positions *(see* 1100a, 1100b, and 1100c) according to a case may be used. The cotter 1100a may be inserted into the case 100 to prevent the user from operating the button member 700 before operating the cotter. By withdrawing the cotter 1100a from the case 100 in the withdrawal direction M1, the button member 700 may be moved upward by the pressing member 1000 or an elastic member (not illustrated) such as a separate spring so that the button member 700 is in the state in which the user can push the button member 700 downward.

A selector hole 110h, 123 through which a selector operation portion 1812 of a selector 1800 is exposed may be formed at the case 100. In the present embodiment, a hole 110h formed at the second case part 100B and a hole 123 formed at the guide part 100C may communicate with each other to form the selector hole 100h, 123. The selector hole 110h, 123 may be formed to be elongated to one side. The selector hole 110h, 123 may be configured to guide the movement path of the selector operation portion 1812.

A switching hole 110g, 124 into which the cotter 1100c is insertable to push a switching member 1900 may be formed at the case 100. In the present embodiment, a hole 110g formed at the first case part 100A and a hole 124 formed at the guide part 100C may communicate with each other to provide the switching hole 110g, 124.

The case 100 may include a pushing guide portion 125 for guiding a reservoir pushing member 500 in the moving direction. The pushing guide portion 125 may include a hole or a groove into which a protrusion 730 of the button member 700 is inserted. The hole or groove of the pushing guide portion 125 may extend in the upper-lower direction to guide the protrusion 730 to move in the upper-lower direction along the groove or hole. In the present embodiment, the pushing guide portion 125 is formed at the guide part 100C of the case 100, and the protrusion 730 is formed at an upper part 700A of the button member 700.

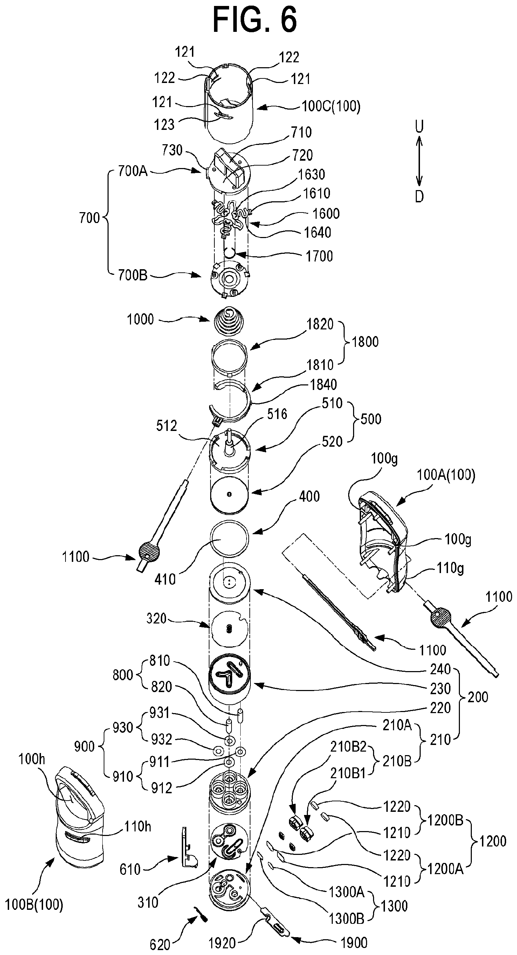

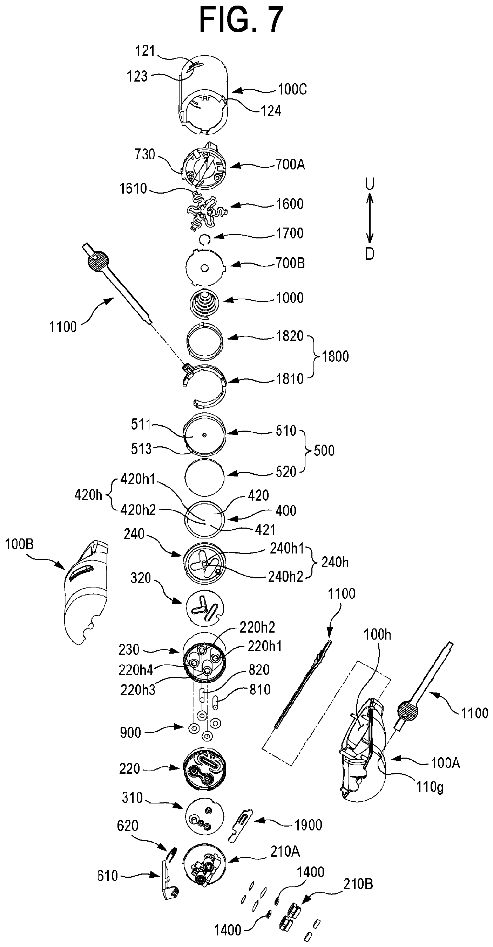

FIGS. 6 and 7 are exploded perspective views of the medicinal liquid control apparatus 80 of FIG. 3. Referring to FIGS. 3 to 7, the medicinal liquid control apparatus 80 may include the case 100. The medicinal liquid control apparatus 80 may include a body 200 that defines at least a portion of the medicinal liquid channel. The medicinal liquid control apparatus 80 may include a valve plate 310. The medicinal liquid control apparatus 80 may include a connection plate 320.

The medicinal liquid control apparatus 80 may include the reservoir 400 that stores a medicinal liquid. The medicinal liquid control apparatus 80 may include the reservoir pushing member 500 configured to push the reservoir 400. The medicinal liquid control apparatus 80 may include a lock bar 610. The medicinal liquid control apparatus 80 may include a lock elastic member 620. The medicinal liquid control apparatus 80 may include the button member 700. The medicinal liquid control apparatus 80 may include at least one medicinal liquid transfer tube 800 that limits a flow rate of a medicinal liquid per hour. The medicinal liquid control apparatus 80 may include at least one sealer 900. The medicinal liquid control apparatus 80 may include the pressing member 1000. The medicinal liquid control apparatus 80 may include the cotter 1100.

The medicinal liquid control apparatus 80 may include at least one air passing filter 1200 that filters out air in the medicinal liquid channel. The medicinal liquid control apparatus 80 may include a hydrophilic boundary filter 1300 disposed on the medicinal liquid channel. The medicinal liquid control apparatus 80 may include a filter spacer 1400.

The medicinal liquid control apparatus 80 may include a support slider 1600. The medicinal liquid control apparatus 80 may include a slider spring 1700. The medicinal liquid control apparatus 80 may include the selector 1800. The medicinal liquid control apparatus 80 may include the switching member 1900.

The button member 700 may be configured to be operated by a user. The medicinal liquid control apparatus 80 may include an elastic member SP, which will be described later, configured to be elastically deformed when the button member 700 is operated.

Referring to FIGS. 3 to 7, the case 100 may include the first case part 100A and the second case part 100B that are coupled to each other. The case 100 may include the guide part 100C. In the present embodiment, the first case part 100A, the second case part 100B, and the guide part 100C are coupled to one another to constitute the case 100, but in another embodiment (not illustrated), various holes corresponding to the holes formed at the guide part 100C may be formed at the first case part 100A and/or the second case part 100B instead, and the case 100 may be implemented without the guide part 100C.

The case 100 may accommodate the body 200 therein. The case 100 may accommodate the sealing plate 300 therein. The case 100 may accommodate the first channel valve 316 therein. The case 100 may accommodate a second channel valve 318 therein. The case 100 may accommodate the reservoir 400 therein. The case 100 may accommodate the reservoir pushing member 500 therein. The case 100 may accommodate the lock bar 610 and the lock elastic member 620 therein. The case 100 may accommodate the pressing member 1000 therein. The case 100 may accommodate the selector 1800 therein. The case 100 may accommodate the components other than the operation part 710 of the button member 700 therein.

A hole 100h into which a user's finger is insertable may be formed at the case 100. The operation part 710 may be exposed in the hole 100h. The cotter 1100 may be inserted into the case 100 to cross the hole 100h in a direction perpendicular to the penetration direction of the hole 100h. A hole 100g into which the cotter 1100 is inserted is formed at the case 100.

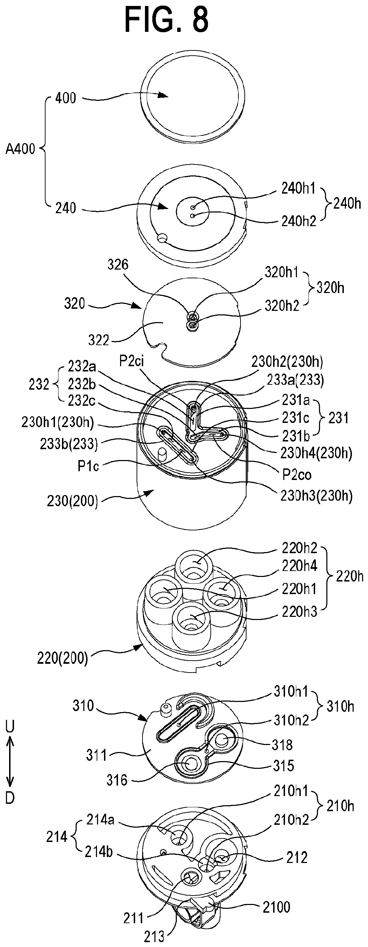

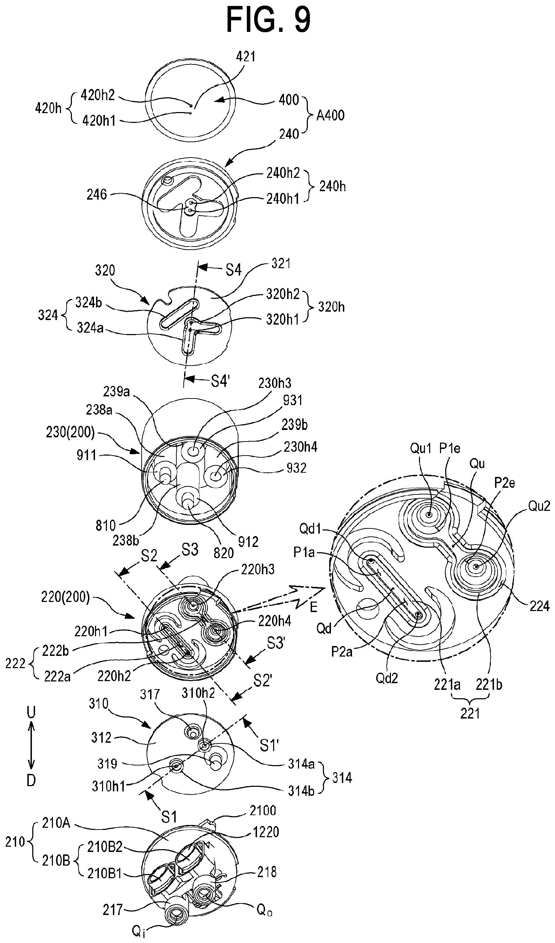

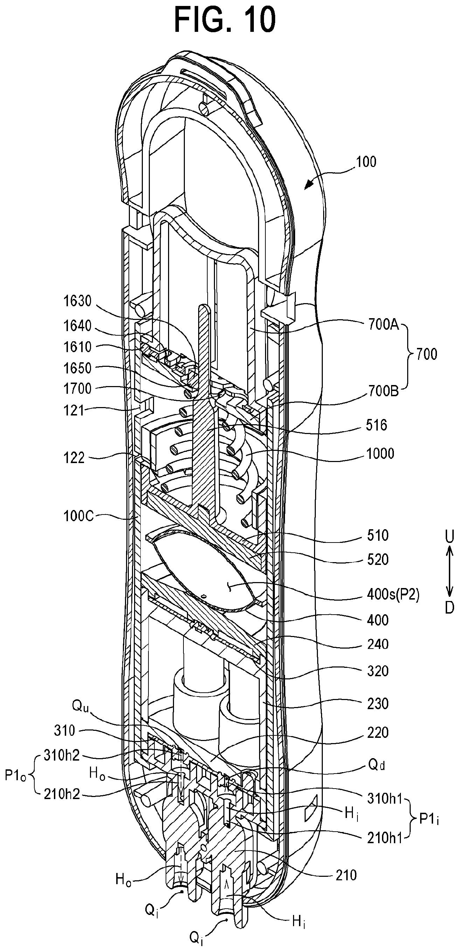

FIGS. 8 and 9 are exploded perspective views illustrating only some of the components of FIG. 6, in which unlike FIG. 6, some components are illustrated in a state of being assembled to one another. FIG. 10 is a perspective view illustrating a cross section of the medicinal liquid control apparatus 80 taken along line S1-S1' of FIG. 9.

Referring to FIGS. 6 to 10, the body 200 may include a plurality of parts 210, 220, 230, and 240. The plurality of parts 210, 220, 230, and 240 may be assembled with one another. The plurality of parts 210, 220, 230, and 240 may be sequentially arranged and assembled with one another. The plurality of parts 210, 220, 230, and 240 may be arranged in the upper-lower direction and assembled with one another.

The body 200 may include a channel forming part 220 that defines at least one extension hole 220h constituting a portion of the medicinal liquid channel. The at least one extension hole 220h may include a plurality of extension holes 220h1, 220h2, 220h3, and 220h4. The body 200 may include a valve support part 210 that supports the lower surface of the valve plate 310. The valve support part 210 and the channel forming part 220 may be coupled to each other.

The body 200 may include a connection part 230 forming at least one extension hole 230h constituting a portion of the medicinal liquid channel. The at least one extension hole 230h may include a plurality of extension holes 230h1, 230h2, 230h3, and 230h4. The body 200 may include a reservoir support part 240 that supports the lower surface of the reservoir 400. The reservoir support part 240 may support the upper surface of the connection plate 320. The connection part 230 and the reservoir support part 240 may be coupled to each other. The channel forming part 220 and the connection part 230 may be coupled to each other.

The valve plate 310 may cover at least a portion of the lower surface of the channel forming part 220. The valve plate 310 may cover at least a portion of the upper surface of the valve support part 210. In the present embodiment, the valve plate 310 may be disposed between the valve support part 210 and the channel forming part 220.

The connection plate 320 may cover at least a portion of the upper surface of the connection part 230. The connection plate 320 may cover at least a portion of the lower surface of the reservoir support part 240. The connection plate 320 may be disposed between the connection part 230 and the reservoir support part 240.

Guide channels (e.g., P1a, P2a, P1e, and P2e) partitioned by the channel forming part 220 and the valve plate 310 may be formed between the channel forming part 220 and the valve plate 310.

For example, the channel forming part 220 may be recessed in the upward direction to form one or more channel grooves 222 extending along the upper surface of the valve plate 310. As the valve plate 310 covers the channel grooves 222 of the channel forming part 220, the above-mentioned guide channels partitioned by the channel forming part 220 and the valve plate 310 may be formed.

As another example, the valve plate 310 may be recessed in the downward direction to form one or more channel grooves (not illustrated) extending along the lower surface of the channel forming part 220. As the channel forming part 220 covers the channel grooves of the valve plate 310, the above-mentioned guide channels partitioned by the channel forming part 220 and the valve plate 310 may be formed.

Guide channels (e.g., P1a, P2a, P1e, and P2e) partitioned by the channel forming part 220 and the valve plate 310 may be formed between the channel forming part 220 and the valve plate 310.

A channel partitioned by the connection part 230 and the connection plate 320 may be formed between the connection part 230 and the connection plate 320. In the present embodiment, the connection part 230 may be recessed in the downward direction to form one or more channel grooves 232 extending along the lower surface of the connection plate 320. As the connection plate 320 covers the channel grooves 232 of the connection part 230, the above-mentioned channels partitioned by the connection part 230 and the connection plate 320 may be formed.

The channel grooves 222 constitute a portion of the medicinal liquid channel by being covered with the valve plate 310. The channel grooves 232 constitute a portion of the medicinal liquid channel by being covered with the connection plate 320.

The channel forming part 220 may include a channel guide rib 221 protruding toward the upper surface of the valve plate 310 and extending along the upper surface of the valve plate 310. The channel guide rib 221 partitions the channel groove 222. The channel guide rib 221 comes into contact with the valve plate 310. As the channel guide rib 221 presses the valve plate 310, the channel formed by the channel groove 222 can be more stably sealed.

The connection part 230 may include a channel guide rib 231 protruding toward the lower surface of the connection plate 320 and extending along the lower surface of the connection plate 320. The channel guide rib 231 partitions the channel groove 232. The channel guide rib 231 comes into contact with the connection plate 320. As the channel guide rib 231 presses the connection plate 320, the channel formed by the channel groove 232 can be more stably sealed.

Points Qd, Qd1, Qd2, Qu, Qu1, and Qu2 located in the medicinal liquid channel will be described with reference to the enlarged view (E) of FIG. 9. An inlet Qi is located at an upstream starting point of the medicinal liquid channel, and an outlet Qo is located at a downstream end point of the medicinal liquid channel. The branching point Qd is a point at which the medicinal liquid flowing along the first channel P1 is split into the second channel P2. The merging point Qu is a point at which the medicinal liquid flowing along the second channel P2 merges into the first channel P1. On the first channel P1, a first split-flow guide point Qd1 is located between the branching point Qd and the merging point Qu. On the second channel P2, a first split-flow guide point Qd1 is located between the branching point Qd and the merging point Qu. On the first channel P1, a first merging guide point Qu1 is located between the branching point Qd and the merging point Qu. On the first channel P1, the first merging guide point Qu1 may be located between the first split-flow guide point Qd1 and the merging point Qu. On the second channel P2, a second merging guide point Qu2 is located between the branching point Qd and the merging point Qu. On the second channel P2, the second merging guide point Qu2 may be located between the second split-flow guide point Qd2 and the merging point Qu. The branching point Qd, the first split-flow guide point Qd1, and the second split-flow guide point Qd2 may be located in a channel groove 222a. The merging point Qu, the first merging guide point Qu1, and the second merging guide point Qu2 may be located in a channel groove 222b.

Referring to FIGS. 6 to 10, the valve support part 210 may define a portion of an inlet channel P1i and a portion of an outlet channel P1o, which will be described later, of the first channel P1. An inlet Qi and an outlet Qo may be formed at the valve support part 210. The valve support part 210 may include the inlet connection portion 217 defining the inlet Qi and the outlet connection portion 218 defining the outlet Qo. The valve support part 210 may include a filter body 210A and a filter cap 210B, which are coupled to each other.

In an embodiment, the boundary filter 1300 may be disposed in the filter body 210A. The filter cap 210B may be disposed in a direction in which one surface of the boundary filter 1300 faces.

In an embodiment, one or more air passing filters 1200 may be disposed on the valve support part 210. The one or more air passing filters 1200 may be fixed to the filter cap 210B. The valve support part 210 may define an air passage branched from the medicinal liquid channel. The valve support part 210 may define at least one vent hole (not illustrated) located at a point where the air passage is connected to the external space. The vent hole may be formed at the filter cap 210B.

The filter cap 210B may include an inlet filter cap 210B1 that defines a vent hole through which air split from the inlet channel P1i is discharged. The filter cap 210B may include an outlet filter cap 210B2 that forms a vent hole through which air split from the outlet channel P1o is discharged.

At least one channel hole 210h constituting a portion of the medicinal liquid channel may be formed at the valve support part 210. The at least one channel hole 210h may include an inlet channel hole 210h1 constituting a portion of the inlet channel P1i. The at least one channel hole 210h may include an outlet channel hole 210h2 constituting a portion of the outlet channel P1o. One end of the inlet channel hole 210h1 defines an inlet Qi, and the other end of the inlet channel hole 210h1 faces the valve plate 310. One end of the outlet channel hole 210h2 defines an outlet Qo, and the other end of the outlet channel hole 210h2 faces the valve plate 310.

An insertion groove 214 into which a protrusion 314 of the valve plate 310 is inserted is formed at the valve support part 210. The insertion groove 214 is formed by being recessed in a direction opposite to the direction facing the valve plate 310. The insertion groove 214 may include an upstream insertion groove 214a in which the other end of the inlet channel hole 210h1 is located. The insertion groove 214 may include a downstream insertion groove 214b in which the other end of the outlet channel hole 210h2 is located.

The valve plate 310 may be in contact with the lower surface of the channel forming part 220. The valve support part 210 may be coupled to the lower side of the channel forming part 220. The medicinal liquid transfer tube 800 may be disposed on the upper portion of the channel forming part 220. The channel forming part 220 may support the lower end of the medicinal liquid transfer tube 800. The channel forming part 220 may support one end of a first medicinal liquid transfer tube 810. The channel forming part 220 may support one end of a second medicinal liquid transfer tube 820.

At least one channel groove 222 may be formed at the channel forming part 220. The at least one channel groove 222 may include at least one of a branching channel groove 222a and a merging channel groove 222b. The branching channel groove 222a may be covered with the valve plate 310 to configure split-flow guide channels P1a and P2a. The merging channel groove 222b may be covered with the valve plate 310 to configure merging guide channels P1e and P2e.

The branching point Qd, the first split-flow guide point Qd1, and the second split-flow guide point Qd2 may be located in the branching channel groove 222a. The branching channel groove 222a may extend in a direction perpendicular to the penetrating direction (upper-lower direction) of a upstream connection hole 310h1 of the valve plate 310. The upper end of the upstream connection hole 310h1 faces the branching point Qd.

The merging point Qu, the first merging guide point Qu1, and the second merging guide point Qu2 may be located in the merging channel groove 222b. The merging channel groove 222b may extend in a direction perpendicular to the penetrating direction (upper-lower direction) of a downstream connection hole 310h2 of the valve plate 310. The upper end of the downstream connection hole 310h2 faces the merging point Qu.

The branching channel groove 222a and the merging channel groove 222b may be disposed to be horizontally spaced apart from each other. The branching channel groove 222a and the merging channel groove 222b may be disposed in parallel to each other.

The channel forming part 220 may include the channel guide rib 221 protruding downward to come into contact with the valve plate 310. The channel guide rib 221 may include a branching channel guide 221a that partitions the branching channel groove 222a. The channel guide rib 221 may include a merging channel guide 221b that partitions the merging channel groove 222b.

The at least one extension hole 220h constituting a portion of the medicinal liquid channel is formed at the channel forming part 220. The extension hole 220h may extend in the upper-lower direction. The lower end of the extension hole 220h may be located in the channel groove 222.

The at least one extension hole 220h may include an extension hole 220h1 constituting a portion of a first upstream extension channel P1b to be described later. The lower end of the extension hole 220h1 may face the first split-flow guide point Qd1.

The at least one extension hole 220h may include an extension hole 220h2 constituting a portion of a second upstream extension channel P2b to be described later. The lower end of the extension hole 220h2 may face the second split-flow guide point Qd2.

The at least one extension hole 220h may include an extension hole 220h3 constituting a portion of a first downstream extension channel P1d to be described later. The lower end of the extension hole 220h3 may face the first merging guide point Qu1.

The at least one extension hole 220h may include an extension hole 220h4 constituting a portion of a second downstream extension channel P2d to be described later. The lower end of the extension hole 220h4 may face the second merging guide point Qu2.

The medicinal liquid transfer tube 800 may be disposed on the lower portion of the connection part 230. The connection part 230 may support the upper end of the medicinal liquid transfer tube 800. The connection part 230 may support the other end of the first medicinal liquid transfer tube 810. The connection part 230 may support the other end of the second medicinal liquid transfer tube 820.

At least one channel groove 232 may be formed at the connection part 230. The at least one channel groove 232 may include at least one of an inlet channel groove 232a, an outlet channel groove 232b, and a connection channel groove 232c. The inlet channel groove 232a may be covered with the connection plate 320 to constitute a portion of the reservoir inlet channel P2ci. The outlet channel groove 232b may be covered with the connection plate 320 to constitute a portion of the reservoir outlet channel P2co. The connection channel groove 232c may be covered with the connection plate 320 to constitute a first connection guide channel P1c.

The inlet channel groove 232a may extend in a direction perpendicular to the penetrating direction (upper-lower direction) of a connection hole 320h1 of the connection plate 320. The lower end of the connection hole 320h1 faces the inlet channel groove 232a.

The outlet channel groove 232b may extend in a direction perpendicular to the penetrating direction (upper-lower direction) of a connection hole 320h2 of the connection plate 320. The lower end of the connection hole 320h2 faces the outlet channel groove 232b.

The lower surface of the connection plate 320 may cover the connection channel groove 232c. The connection plate 320 may cover the entire connection channel groove 232c so that the connection channel groove 232c does not have an upwardly connected channel.

The inlet channel groove 232a and the outlet channel groove 232b may be disposed to be horizontally spaced apart from each other. The inlet channel groove 232a, the outlet channel groove 232b, and the connection channel groove 232c may be disposed to be horizontally spaced apart from each other.

The connection part 230 may include the channel guide rib 231 protruding downward to come into contact with the connection plate 320. The channel guide rib 231 may include an inlet channel guide 231a that partitions the inlet channel groove 232a. The channel guide rib 231 may include an outlet channel guide 231b that partitions the outlet channel groove 232b. The channel guide rib 231 may include a connection channel guide 231c that partitions the connection channel groove 232c.

The at least one extension hole 230h constituting a portion of the medicinal liquid channel may be formed at the connection part 230. The extension hole 230h may extend in the upper-lower direction. The upper end of the extension hole 230h is located in the channel groove 232.

The at least one extension hole 230h may include an extension hole 230h1 constituting a portion of a first upstream extension channel P1b to be described later, and an extension hole 230h2 constituting a portion of a second upstream extension channel P2b to be described later. The upper end of the extension hole 230h1 and the upper end of the extension hole 230h2 may be located in the connection channel groove 232c.

The at least one extension hole 230h may include an extension hole 230h3 constituting a portion of a first downstream extension channel P1d to be described later, and an extension hole 230h4 constituting a portion of a second downstream extension channel P2d to be described later. The upper end of the extension hole 230h3 may be located in the inlet channel groove 232a. The upper end of the extension hole 230h4 may be located in the outlet channel groove 232b.

The extension hole 220h1 of the channel forming part 220, a capillary channel 810p of the first medicinal liquid transfer tube 810, and the extension hole 230hl of the connection part 230 may be sequentially connected to each other to define the first upstream extension channel P1b. The extension hole 220h2 of the channel forming part 220, a capillary channel 820p of the second medicinal liquid transfer tube 820, and the extension hole 230h2 of the connection part 230 may be sequentially connected to each other to define the second upstream extension channel P2b. The extension hole 230h3 of the connection part 230 and the extension hole 220h3 of the channel forming part 220 may be sequentially connected to each other to define the first downstream extension channel P1d. The extension hole 230h4 of the connection part 230 and the extension hole 220h4 of the channel forming part 220 may be sequentially connected to each other to define the first downstream extension channel P1d.

At least one of the channel forming part 220 and the connection part 230 may include transfer tube seating portions 238a and 238b on which the medicinal liquid transfer tube 800 is seated. The transfer tube seating portions 238a and 238b may include a first transfer tube seating portion 238a on which the first medicinal liquid transfer tube 810 is seated, and a second transfer tube seating portion 238b on which the second medicinal liquid transfer tube 820 is seated. The transfer tube seating portions 238a and 238b may define a hole into which the medicinal liquid transfer tube 800 is inserted, and the hole into which the medicinal liquid transfer tube 800 is inserted may communicate with the above-described extension hole. In the present embodiment, the transfer tube seating portions 238a and 238b are formed at the connection part 230, wherein the extension hole 230h1 communicates with the hole of the first transfer tube seating portion 238a, and the extension hole 230h2 communicates with the second transfer tube seating portion 238b.

At least one of the channel forming part 220 and the connection part 230 may include channel forming portions 239a and 239b extending the above-described extension holes in the upper-lower direction. The channel forming portions 239a and 239b may extend in parallel to the transfer tube seating portion 238a. In the present embodiment, the channel forming portions 239a and 239b are formed at the connection part 230, wherein the first channel forming part 239a extends the extension hole 230h1 in the upper-lower direction, and the second channel forming portion 239b extends the extension hole 230h4 in the upper-lower direction.

A rib groove 233 into which a protrusion 324 is inserted may be formed at the connection part 230. A rib groove 233a into which a protrusion 324a is inserted and a rib groove 233b into which a protrusion 324b is inserted may be formed at the connection part 230. The rib groove 233 may extend along the circumference of the channel guide rib 231. The rib groove 233 may be recessed downward.

The sealer 900 may be sandwiched between the channel forming part 220 and the connection part 230. The channel forming part 220 and the connection part 230 may be coupled to each other such that the medicinal liquid transfer tube 800 is disposed therein.

The reservoir support part 240 may define a portion of a second connection guide channel P2c to be described later. A connection hole 240h may be formed at the reservoir support part 240. The connection hole 240h may penetrate the reservoir support part 240 in the upper-lower direction. The connection hole 240h may be formed at the central portion of the reservoir support part 240.

The connection hole 240h may include an inlet connection hole 240h1 and an outlet connection hole 240h2. The inlet connection hole 240h1 and the outlet connection hole 240h2 constitute a portion of the second channel P2.

The reservoir support part 240 may be formed in a plate shape. The reservoir support part 240 may be formed in a circular shape as a whole when viewed from above.

A groove 246 into which an upper protrusion 326 is inserted may be formed at the reservoir support part 240. The groove 246 may be recessed upward.

The medicinal liquid channel may penetrate the valve plate 310. A connection hole 310h, which is connected to the channel groove 222 and constitutes a portion of the medicinal liquid channel, may be formed at the valve plate 310. The connection hole 310h may penetrate the valve plate 310 in the upper-lower direction.

The medicinal liquid channel may penetrate the connection plate 320. A connection hole 320h, which is connected to the channel groove 232 and constitutes a portion of the medicinal liquid channel, may be formed at the connection plate 320. The connection hole 320h may penetrate the connection plate 320 in the upper-lower direction.

The channel hole 210h of the valve support part 210, the connection hole 310h of the valve plate 310, and the guide channel partitioned by the channel forming part 220 and the valve plate 310 may be sequentially connected to constitute a portion of the medicinal liquid channel. In addition, the extension hole 220h of the channel forming part 220 and the guide channel may be connected to constitute a portion of the medicinal liquid channel.

The connection hole 240h of the reservoir support part 210, the connection hole 320h of the connection plate 320, and the channel partitioned by the connection part 230 and the connection plate 320 may be sequentially connected to constitute a portion of the medicinal liquid channel. In addition, the extension hole 230h of the connection part 230 and the channel partitioned by the connection part 230 and the connection plate 320 may be connected to constitute a portion of the medicinal liquid channel.

The valve plate 310 may be formed in a plate shape. The valve plate 310 may be formed in a circular shape when viewed from above. The material of the valve plate 310 may be more flexible than the material of the channel forming part 220. In addition, the material of the valve plate 310 may be more flexible than the material of the valve support part 210. In the present disclosure, "more flexible" means "smaller in elastic modulus." In the present embodiment, the material of the channel forming part 220 and the valve support part 210 includes a synthetic resin, and the material of the valve plate 310 includes rubber or silicone.

The valve plate 310 may include a cover surface 311 that is in contact with the channel forming part 220. The valve plate 310 may include an auxiliary cover surface 312 that is in contact with the valve support part 210. The cover surface 311 may form the upper surface, and the auxiliary cover surface 312 may form the lower surface.

The upstream connection hole 310h1 connected to the branching channel groove 222a and the downstream connection hole 310h2 connected to the merging channel groove 222b may be formed at the valve plate 310. The inlet channel hole 210h1 of the valve support part 210 and the upstream connection hole 310h1 may be sequentially connected to each other to define the inlet channel P1i *(see* FIG. 10). The downstream connection hole 310h2 and the outlet channel hole 210h2 of the valve support part 210 may be sequentially connected to each other to define the outlet channel P1o (*see* FIG. 10).

The valve plate 310 may include at least one protrusion 314 protruding from the auxiliary cover surface 312 to a direction facing the valve support part 210. The lower end of the connection hole 310h may be located at the lower end of the protrusion 314. The at least one protrusion 314 may include an upstream protrusion 314a corresponding to the upstream connection hole 310h1 and a downstream protrusion 314b corresponding to the downstream connection hole 310h2.

The valve plate 310 may include a sealing rib 315 protruding upward to surround the channel guide rib 221. The sealing rib 315 may surround the merging channel guide 221b. The valve plate 310 may include a sealing rib (not illustrated) protruding upward to surround the branching channel guide 221a. The lower surface of the channel forming part 220 may be recessed upward to provide a sealing groove 224 into which the sealing rib 315 is inserted.

The connection plate 320 may be formed in a plate shape. The connection plate 320 may be formed in a circular shape when viewed from above. The connection plate 320 may be made of a material that is more flexible than the material of the connection part 230. In addition, the connection plate 320 may be made of a material that is more flexible than the material of the reservoir support part 240. In the present embodiment, the material of the connection part 230 and the reservoir support part 240 includes a synthetic resin, and the material of the connection plate 320 includes rubber or silicon.

The connection plate 320 may include a cover surface 321 that is in contact with the connection part 230. The connection plate 320 may include an auxiliary cover surface 322 that is in contact with the reservoir support part 240. The cover surface 321 may form the lower surface, and the auxiliary cover surface 312 may form the upper surface.

A first connection hole 320h1, which constitutes a portion of the second channel P2 and is connected to the inlet connection hole 240h1 of the reservoir support part 240, may be formed at the connection plate 320. A second connection hole 320h2, which constitutes a portion of the second channel P2 and is connected to the outlet connection hole 240h2 of the reservoir support part 240, may be formed at the connection plate 320. The lower end of the first connection hole 320h1 may face the inlet channel groove 232a of the connection part 230, and the lower end of the second connection hole 320h2 may face the outlet channel groove 232b of the connection part 230.

The connection plate 320 may include the protrusion 324 protruding downward to surround the channel guide rib 231. The protrusion 324 may include the protrusion 324a surrounding the inlet channel guide 231a and the outlet channel guide 231b and the protrusion 324b surrounding the connection channel guide 231c. The connection plate 320 may include the upper protrusion 326 that protrudes upward and extends along the circumference of the connection hole 320h.

A reservoir assembly A400 is a component including the assembly of the reservoir 400 and the reservoir support part 240. The medicinal liquid control apparatus 80 may include the reservoir assembly A400.

The reservoir (reservoir) 400 defines the internal space 400s for storing a medicinal liquid. In the medicinal liquid control apparatus 80, the internal space 400s of the reservoir 400 is located on the medicinal liquid channel. In the medicinal liquid control apparatus 80, the internal space 400s of the reservoir 400 may be located on a second channel P2.

The reservoir 400 is configured such that the volume of the internal space 400s can be changed. As the volume of the internal space 400s of the reservoir 400 increases, the medicinal liquid may be stored, and as the volume of the internal space 400s of the reservoir 400 decreases, the stored medicinal liquid may be discharged.

The reservoir 400 may include a first film 410 of an upper side and a second film 420 of a lower side coupled to each other. For example, each of the first film 410 and the second film 420 may be a polyvinyl chloride (PVC) film. The first film 410 and the second film 420 may be coupled at the top and bottom of respective edges thereof to define the internal space 400s between the first film 410 and the second film 420.

At one portion 421 of the outer surface of the reservoir 400, at least one hole 420h connected to the internal space 400s may be formed. The at least one hole 420h may include an inlet hole 420h1 and an outlet hole 420h2. The inlet hole 420h1 may constitute a portion of the reservoir inlet channel P2ci, and the outlet hole 420h2 may constitute a portion of the reservoir outlet channel P2co.

The one portion 421 of the reservoir 400 may be fixed to the reservoir support part 240. The one portion 421 may be located in the central portion of the reservoir 400. The one portion 421 may be located in the center of the lower surface of the reservoir 400. The one portion 421 may be located on the second film 420.

The reservoir support part 240 may be disposed below the reservoir 400. The reservoir 400 may be fixed to the upper surface of the reservoir support part 240. The upper surface of the reservoir support part 240 may include an upwardly convex surface.

The one portion 421 of the reservoir 400 may be fixed to one portion of the upper surface of the reservoir support part 240. The one portion of the reservoir support part 240 may be located in a central portion of the reservoir support part 240. The one portion 421 of the reservoir 400 may be located in the center of the lower surface of the reservoir 400, and the convex surface of the reservoir support part 240 may protrude from a position corresponding to the center of the reservoir 400.

The inlet connection hole 240h1, which constitutes a portion of the medicinal liquid channel and is connected to the inlet hole 420h1 of the reservoir 400, may be formed at the reservoir support part 240. The outlet connection hole 240h2, which constitutes a portion of the medicinal liquid channel and is connected to the outlet hole 420h2, may be formed at the reservoir support part 240.

The inlet connection hole 240h1 may constitute a portion of the reservoir inlet channel P2ci. The inlet connection hole 240h1 may be connected to the inlet hole 420h1 of the reservoir 400. The inlet hole 420h1 and the inlet connection hole 240h1 may be vertically connected to each other. The inlet connection hole 240h1 may be connected to the first connection hole 320h1 of the connection plate 320. The inlet connection hole 240h1 and the first connection hole 320h1 may be vertically connected to each other.

The outlet connection hole 240h2 may constitute a portion of the reservoir outlet channel P2co. The outlet connection hole 240h2 may be connected to the outlet hole 420h2 of the reservoir 400. The outlet hole 420h2 and the outlet connection hole 240h2 may be vertically connected to each other. The outlet connection hole 240h2 may be connected to the second connection hole 320h2 of the connection plate 320. The outlet connection hole 240h2 and the second connection hole 320h2 may be vertically connected to each other.

The reservoir pushing member 500 is configured to be capable of pressing the reservoir 400. The reservoir pushing member 500 may be disposed above the reservoir 400. The reservoir pushing member 500 may be configured to be capable of pressing the reservoir 400 downward. When the reservoir pushing member 500 moves downward, the reservoir 400 is pushed between the reservoir pushing member 500 and the reservoir support part 240, so that the medicinal liquid in the internal space 400s of the reservoir 400 may be discharged to the outside of the reservoir 400.

The reservoir pushing member 500 may include an upper plate 510 and a reservoir pressing portion 520. The reservoir pushing member 500 may include a slide protrusion 516 protruding upward. The slide protrusion 516 may protrude upward from the center of the reservoir pushing member 500.

The upper plate 510 may be coupled to the upper side of the reservoir pressing portion 520. The upper plate 510 may be formed in a circular shape as a whole when viewed from above. The upper plate 510 may include a coupling surface 511 coupled to the upper surface of the reservoir pressing portion 520, and a pushing surface 512 pressed by the pressing member 1000. The upper plate 510 may include a side guide 513 that extends along the circumference of the coupling surface 511 to be in contact with the edge of the reservoir pressing portion 520. The slide protrusion 516 may be formed at the upper plate 510. The slide protrusion 516 is configured to be slidable in the upper-lower direction with respect to the support slider 1600.

The reservoir pressing portion 520 includes a lower surface that presses the reservoir 400. The reservoir pressing portion 520 may be formed of a material that is more flexible than that of the upper plate 510. In the present embodiment, the material of the upper plate 510 may include a synthetic resin, and the material of the reservoir pressing portion 520 may include rubber or silicone.

The lock bar 610 is configured to be movable in the upper-lower direction with respect to the case 100. The lock elastic member 620 is configured to press the lock bar 610 upward. The lock elastic member 620 may be configured to be elastically deformed when the lock bar 610 moves downward. The lock elastic member 620 may be disposed on the body 200. The lock elastic member 620 may support the lock bar 610.

The lock elastic member 620 may use one of various known manners of exerting an elastic force. For example, the lock elastic member 620 may include various types of members such as a compression spring and an air spring, or may include a member made of a material such as rubber to be elastically compressed.

The button member 700 is configured to be movable downward. The button member 700 may be configured to be movable in the upper-lower direction.

In the present embodiment, the button member 700 is disposed to be spaced apart upward from the upper side of the reservoir pushing member 500. The button member 700 is configured to be capable of pressing the upper end of the pressing member 1000 downward. In this embodiment, when the user pushes the operation part 710 of the button member 700 downward, the button member 700 pushes the pressing member 1000 downward, and the pressing member 1000 pushes the reservoir pushing member 500 downward.

In another embodiment (not illustrated), the button member 700 may be configured to be spaced apart upward from the reservoir pushing member 500, and to be capable of pressing the reservoir pushing member 500 downward. In the present embodiment, when the user pushes the operation part 710 of the button member 700 downward, the button member 700 may directly push the reservoir pushing member 500 downward.

The button member 700 may include an upper surface portion 720 that covers the upper side of the support slider 1600. The button member 700 may include the operation part 710 protruding upward from the upper surface portion 720.

The pressing member 1000 is disposed between the button member 700 and the reservoir pushing member 500. The pressing member 1000 may be configured to press the reservoir pushing member 500 downward.

In the present embodiment, the pressing member 1000 is configured such that the lower end of the pressing member 1000 presses the reservoir pushing member 500 downward while the pressing member 1000 is compressively and elastically deformed in the upper-lower direction when the upper end of the pressing member 1000 is pushed downward by the button member 700. In the present embodiment, the pressing member 1000 is configured to be elastically deformed when the button member 700 is operated. In the present embodiment, the pressing member 1000 is configured to be elastically deformed when the reservoir pushing member 500 and the button member 700 come close to each other. The pressing member 1000 may use one of various known manners of exerting an elastic force. For example, the pressing member 1000 may include various types of members such as a compression spring, a torque spring, and an air spring, or may include a member made of a material such as rubber to be elastically compressed.

In another embodiment (not illustrated), the pressing member may not be a spring or the like, but may have a configuration like a bar extending in the upper-lower direction to move downward together with the button member to push the reservoir pushing member 500 downward. In this embodiment, the pressing member may be configured integrally with or coupled to the button member.

The medicinal liquid control apparatus 80 may include the at least one medicinal liquid transfer tube 800 having capillary channels 810p and 820p constituting a portion of the medicinal liquid channel. The medicinal liquid transfer tube 800 is coupled to the body 200. The medicinal liquid transfer tube 800 may have a function of limiting the flow rate of the medicinal liquid per hour. As an example, the medicinal liquid transfer tube 800 may include a capillary tube. As another example, the medicinal liquid transfer tube 800 may include a polymer microtube. In addition, the medicinal liquid transfer tube 800 may be configured in various shapes having a capillary channel using various materials. For example, the capillary channel 820p may have a diameter of about 0.04 to 0.08 mm, thereby limiting the flow rate of a medicinal liquid per hour.

The at least one medicinal liquid transfer tube 800 may include the first medicinal liquid transfer tube 810 that defines the capillary channel 810p constituting a portion of the first channel P1. The first medicinal liquid transfer tube 810 may be disposed in at least one of the plurality of parts 210, 220, 230, and 240. In the present embodiment, the capillary channel 810p constitutes a portion of the first upstream extension channel P1b, but in another embodiment (not illustrated), the capillary channel 810p may constitute a portion of the first downstream extension channel P1d.

The at least one medicinal liquid transfer tube 800 may include the second medicinal liquid transfer tube 820 that defines the capillary channel 820p constituting a portion of the second channel P2. The second medicinal liquid transfer tube 820 may be disposed in at least one of the plurality of parts 210, 220, 230, and 240. In the present embodiment, the capillary channel 820p constitutes a portion of the second upstream extension channel P2b, but in another embodiment (not illustrated), the capillary channel 820p may constitute a portion of the second downstream extension channel P2d.

At least a portion of the medicinal liquid transfer tube 800 may be in contact with the inner surface of the body 200. The medicinal liquid transfer tube 800 may be disposed to pass through a sealer 910.

The sealer 900 may be formed in a ring shape. The sealer 900 may be formed of an elastic material such as rubber. In the present embodiment, the sealer 900 is disposed to be sandwiched between the channel forming part 220 and the channel forming part 220.

The at least one sealer 900 may include at least one transfer tube sealer 910 disposed between the outer surface of the medicinal liquid transfer tube 800 and the inner surface of the housing 81. The transfer tube sealer 910 may block the medicinal liquid from flowing between the outer surface of the medicinal liquid transfer tube 800 and the inner surface of the body 200. The transfer tube sealer 910 may surround the circumference of the medicinal liquid transfer tube 800. The at least one transfer tube sealer 910 may include a first transfer tube sealer 911 disposed between the outer surface of the first medicinal liquid transfer tube 810 and the inner surface of the body 200. The at least one transfer tube sealer 910 may include a second transfer tube sealer 912 disposed between the outer surface of the second medicinal liquid transfer tube 820 and the inner surface of the body 200.

The at least one sealer 900 may include at least one connection sealer 930 that is sandwiched between two parts 220 and 230 which are coupled to each other to prevent leakage of the medicinal liquid between the two parts 220 and 230. The connection sealer 930 is disposed in a portion of the medicinal liquid channel in which the medicinal liquid transfer tube 800 is not disposed. The at least one connection sealer 930 may include a first connection sealer 931 disposed in the first downstream extension channel P1d defined by the two parts 220 and 230 that are coupled to each other. The at least one connection sealer 930 may include a second connection sealer 932 disposed in the second downstream extension channel P2d defined by the two parts 220 and 230 which are coupled to each other.

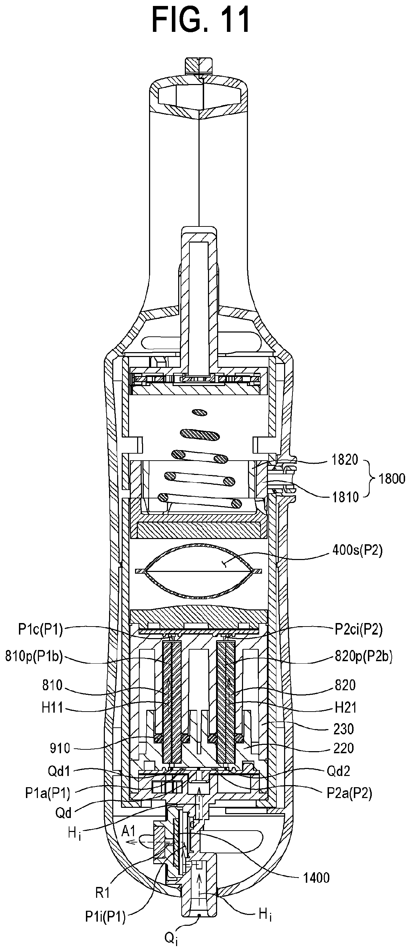

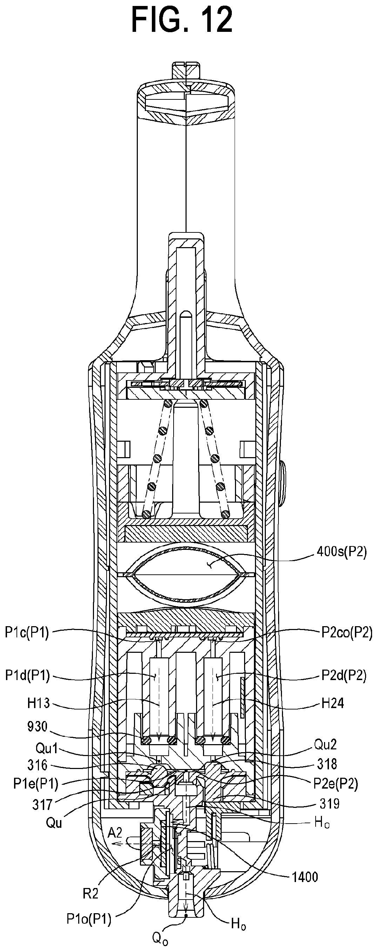

The medicinal liquid control apparatus 80 may include at least one hydrophobic air passing filter 1200. The air passing filter 1200 blocks the passage of the medicinal liquid but allows air to pass therethrough. The air passing filter 1200 may be disposed on a filter support part 210. The filter support part 210 may define a passage R1 of air split from the inlet channel P1i *(see* FIG. 11). The filter support part 210 may define a passage R2 of air split from the outlet passage P1o *(see* FIG. 12). Arrow A1 in FIG. 11 illustrates a direction in which air passes along the passage R1, and arrow A2 in FIG. 12 illustrates a direction in which air passes along the passage R2.

The at least one air passing filter 1200 may include an upstream air passing filter 1200A located upstream of the branching point Qd on the first channel P1. The upstream air passing filter 1200A may be configured to discharge air in the first channel P1 to the outside. The upstream air passing filter 1200A may include a first air passing filter 1210 disposed at a boundary between the air passage R1 and the inlet channel P1i. The upstream air passing filter 1200A may further include a second air passing filter 1220 disposed on the air passage R1.

The at least one air passing filter 1200 may include a downstream air passing filter 1200B located downstream of the merging point Qu on the first channel P1. The downstream air passing filter 1200B may be configured to discharge air in the first channel P1 to the outside. The downstream air passing filter 1200B may include a first air passing filter 1210 disposed at a boundary between the air passage R2 and the outlet channel P1o. The upstream air passing filter 1200A may further include the second air passing filter 1220 disposed on the air passage R2.

The air passing filter 1200 may include the first air passing filter 1210 and the second air passing filter 1220 that are sequentially disposed on the air passages R1 and R2. The second air passing filter 1220 is configured to allow air that has passed through the first air passing filter 1210 to pass therethrough. The second air passing filter 1220 may perform a function of preventing the internal medicinal liquid from flowing out even when a pore, a bonded portion, or the like of the first air passing filter 1210 is damaged.

The second air passing filter 1220 may be produced by processing the same material as the first air passing filter 1210 or a porous plastic material. As an example, the second air passing filter 1220 may be configured such that a hydrophobic porous plastic resin material fills cross-sectional areas of some of the air passages R1 and R2. For example, the material of the second air passing filter 1220 is available from Porex Corporation (web site: https://www.porex.com) of Fairburn, GA 30213, USA. A product released under the name of Porex Hydrophobic Vents from Porex Corporation is available, wherein the product is made of a material of polyethyl polytetrafluoroethylene.

The medicinal liquid control apparatus 80 may include at least one hydrophilic boundary filter 1300 disposed on the medicinal liquid channel. The boundary filter 1300 may be disposed to cross the medicinal liquid channel. The at least one boundary filter 1300 may include an upstream boundary filter 1300A disposed between the upstream air passing filter 1200A and the branching point Qd on the inlet channel P1i. The at least one boundary filter 1300 may include a downstream boundary filter 1300B disposed between the outlet Qo and the downstream air passing filter 1200B on the outlet channel P1o.

The boundary filter 1300 is configured to act as a pressure interface between the upstream channel portion and the downstream channel portion with reference to the boundary filter 1300 when the boundary filter is wetted with the medicinal liquid. By the boundary filter 1300, the upstream channel portion and the downstream channel portion may have different internal pressures. For example, the boundary filter 1300 may be configured in at least one of a mesh structure and a fiber structure. The boundary filter 1300 may additionally have a function of filtering impurities.

The filter spacer 1400 may separate the air passing filter 1200 from the inner surface that partitions the air passage of the filter cap 210B. The filter spacer 1400 may be inserted into the filter cap 210B.

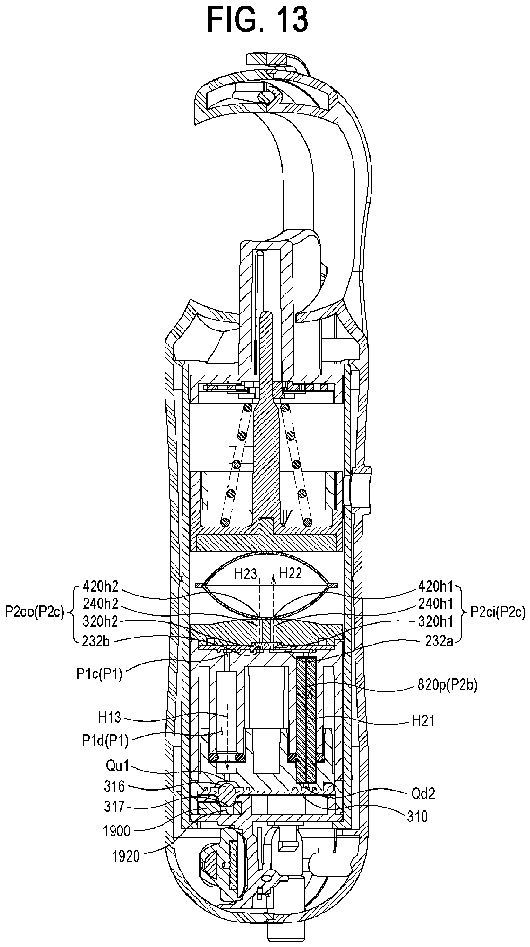

FIG. 11 is a cross-sectional view of the medicinal liquid control apparatus 80 taken along line S2-S2' in FIG. 9. FIG. 12 is a cross-sectional view of the medicinal liquid control apparatus 80 taken along line S3-S3' in FIG. 9. FIG. 13 is a cross-sectional view of the medicinal liquid control apparatus 80 taken along line S4-S4' in FIG. 9.

Referring to FIGS. 10 to 13, the inlet channel P1i, the first split-flow guide channel P1a, the first upstream extension channel P1b, the first connection guide channel P1c, the first downstream extension channel P1d, the first merging guide channel P1e, and the outlet passage P1o may be sequentially connected to each other to form the first channel P1. The first capillary channel 810p may form a portion of the first upstream extension channel P1b.

The second split-flow guide channel P2a, the second upstream extension channel P2b, the reservoir inlet channel P2ci, the internal space 400s of the reservoir 400, the reservoir outlet channel P2co, the second downstream extension channel P2d, and the second merging guide channel P2e may be sequentially connected to each other to form the second channel P2. The second capillary channel 820p may constitute a portion of the second upstream extension channel P2b.

Referring to FIGS. 10 and 11, the inlet channel P1i is a channel section from the inlet Qi to the branching point Qd. After flowing along the inlet channel P1i *(see* arrow Hi), the medicinal liquid is split at the branching point Qd and flows along the split-flow guide channels P1a and P2a. The first split-flow guide channel P1a is a channel section from the branching point Qd to the first split-flow guide point Qd1. After flowing along the first split-flow guide channel P1a, the medicinal liquid flows along the first upstream extension channel P1b *(see* arrow H11). The second split-flow guide channel P2a is a channel section from the branching point Qd to the second split-flow guide point Qd2. After flowing along the second split-flow guide channel P2a, the medicinal liquid flows along the second upstream extension channel P2b *(see* arrow H21).

Referring to FIGS. 11 and 12, the first connection guide channel P1c may interconnect the downstream end of the first upstream extension channel P1b and the upstream end of the first downstream extension channel P1d. After sequentially flowing along the first upstream extension channel P1b and the first connection guide channel P1c, the medicinal liquid flows along the first downstream extension channel P1d (arrow H13).

Referring to FIGS. 12 and 13, the second connection guide channel P2c may interconnect the downstream end of the second upstream extension channel P2b and the downstream end of the second downstream extension channel P2d. After sequentially flowing along the second upstream extension channel P2b and the second connection guide channel P2c, the medicinal liquid flows along the second downstream extension channel P2d (arrow H24).

Referring to FIG. 13, the inlet channel groove 232a of the connection part 230, the first connection hole 320h1 of the connection plate 320, the inlet connection hole 240h1 of the reservoir support part 240, and the inlet hole 420h1 of the reservoir 400 may be sequentially connected to each other to form the reservoir inlet channel P2ci.

Referring to FIGS. 12 and 13, the outlet hole 420h2 of the reservoir 400, the outlet connection hole 240h2 of the reservoir support part 240, the second connection hole 320h2 of the connection plate 320, and the outlet channel groove 232b of the connection part 230 may be sequentially connected to each other to provide the reservoir outlet channel P2co. The medicinal liquid in the internal space 400s of the reservoir 400 flows along the reservoir outlet channel P2co *(see* arrow H23), and then flows along the second downstream extension channel P2d *(see* arrow H24).

Referring to FIG. 12, the split flows of the medicinal liquid split by the split-flow guide channels P1a and P2a are merged through the merging guide channels P1e and P2e to flow along the outlet channel P1o *(see* arrow Ho). The first merging guide channel P1e is a channel section from the first merging guide point Qu1 to the merging point Qu. After flowing along the first downstream extension channel P1d *(see* arrow H13), the medicinal liquid flows along the first merging guide channel P1e. In addition, the second merging guide channel P2e is a channel section from the second merging guide point Qu2 to the merging point Qu. After flowing along the second downstream extension channel P2d *(see* arrow H24), the medicinal liquid flows along the second merging guide channel P2e. The outlet channel P1o is a channel section from the merging point Qu to the outlet Qo. After flowing along the merging guide channels P1e and P2e, the medicinal liquid flows along the outlet channel P1o *(see* arrow Ho).

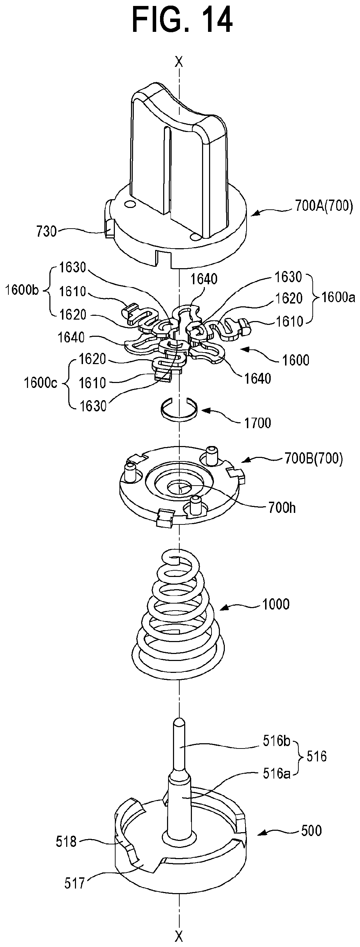

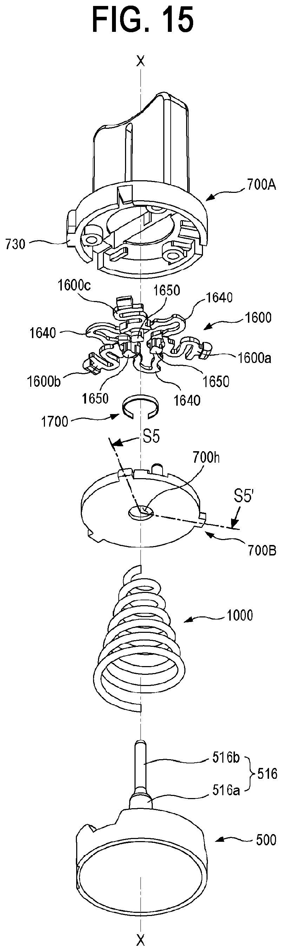

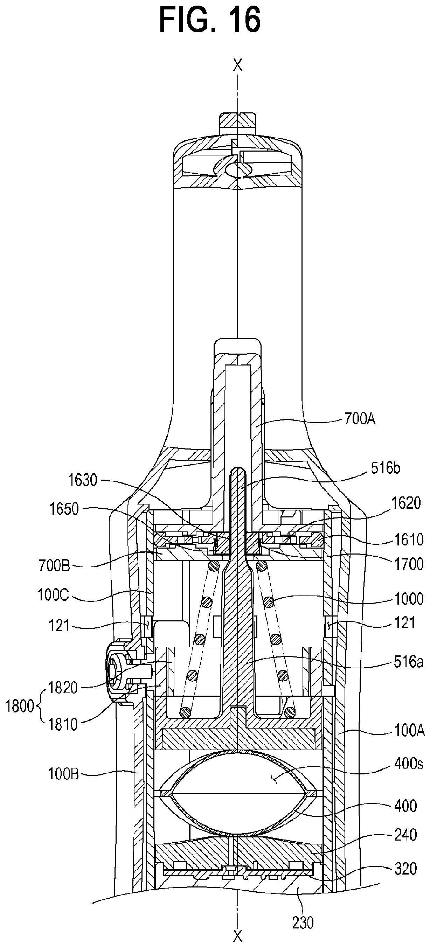

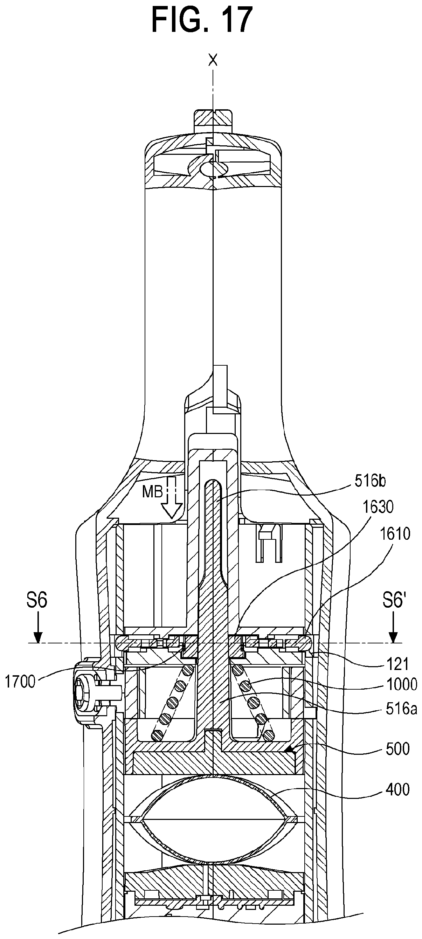

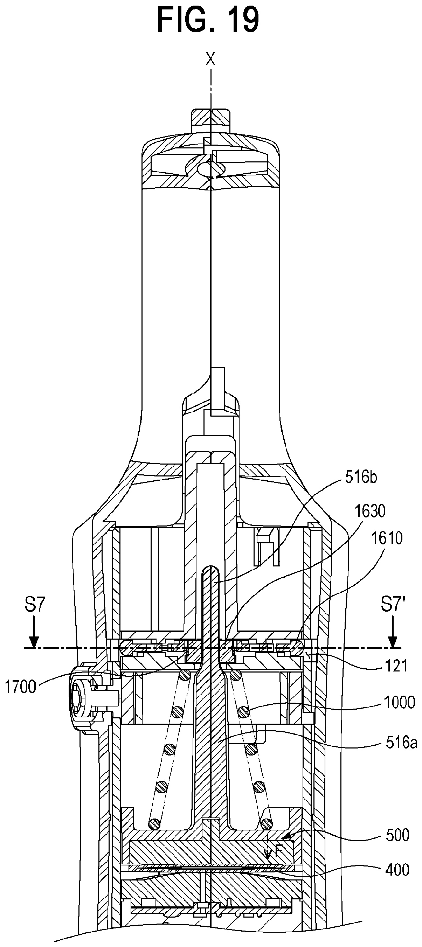

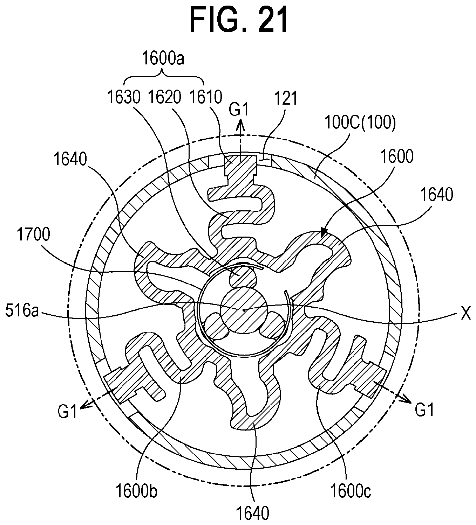

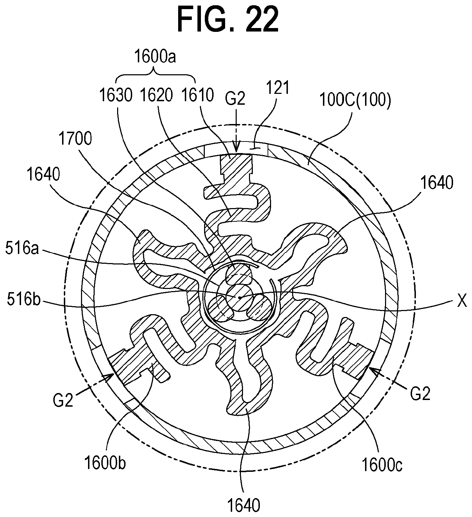

FIGS. 14 and 15 are exploded perspective views of the button member 700, the reservoir pushing member 500, the pressing member 1000, the support slider 1600, and the slider spring 1700 of FIG. 6. FIGS. 16 to 20 are longitudinal cross-sectional views of the medicinal liquid control apparatus 80 taken along line S5-S5' of FIG. 15, illustrating the operation mechanism of the button member 700, the reservoir pushing member 500, the pressing member 1000, the support slider 1600, and the slider spring 1700. FIG. 21 is a cross-sectional view of the medicinal liquid control apparatus 80 taken along line S6-S6' of FIG. 17. FIG. 22 is a cross-sectional view of the medicinal liquid control apparatus 80 taken along line S7-S7' in FIG. 19.

In the description of the medicinal liquid control apparatus 80, a circumferential direction centered on an imaginary central axis X extending in the upper-lower direction is defined as a "circumferential direction," a direction away from the central axis X may be defined as a "radially outward direction," and a direction approaching the central axis X may be defined as a "radially inward direction".

Referring to FIGS. 14 to 22, the medicinal liquid control apparatus 80 may be configured such that the elastically compressed pressing member 1000 pushes the reservoir pushing member 500 to move downward even after the downward movement of the button member 700 has been completed. Even if the user does not continuously push the button member 700 until all the medicinal liquid in the reservoir 400 is discharged, the pressing member 1000 pushes the reservoir pushing member 500 until all the medicinal liquid in the reservoir 400 is discharged. The cotter 1100a may be detachably coupled to the case in the state in which the button member 700 is pushed (*see* FIG. 3). The medicinal liquid control apparatus 80 according to an embodiment may include the reservoir 400, the button member 700, the support slider 1600, the slider spring 1700, the pressing member 1000, and the reservoir pushing member 500.

An engagement groove 121 into which an engagement protrusion 1610 of the support slider 1600 is insertable may be formed at the case 100. In the present embodiment, the engagement groove 121 is formed at the guide part 100C. The engagement protrusion 1610 may be engaged with the engagement groove 121. A plurality of engagement grooves 121 corresponding to a plurality of engagement protrusions 1610 may be formed at the guide part 100C.

The guide part 100C may extend along the circumference of the button member. The guide part 100C may extend along the circumference of the reservoir pushing member 500. The guide part 100C may be configured to guide the moving direction of the button member 700. The guide part 100C may be configured to guide the moving direction of the reservoir pushing member 500.

The support slider 1600 may be disposed on the button member 700. The support slider 1600 may be configured to move upward and downward together with the button member 700. The support slider 1600 may be formed of, for example, a plastic material.

The support slider 1600 may include at least one engagement protrusion 1610 protruding to an engagement direction G1 transverse to the upper-lower direction *(see* FIG. 21). In the present disclosure, a release direction G2 may be defined as a direction opposite to the engagement direction G1 (*see* FIG. 22). In the case of the support slider 1600 including the plurality of engagement protrusions 1610, the engagement direction G1 and the release direction G2 corresponding to each engagement protrusion may be different from each other. In the present embodiment, the engagement direction G1 is a radially outward direction, but is not limited thereto, and the engagement direction may be set to a direction other than the radially outward direction according to embodiments.

The engagement protrusions 1610 may be configured to be inserted into the engagement grooves 121 when the button member 700 moves downward to reach a predetermined position (*see* FIGS. 17 and 21). The support slider 1600 is configured to be engaged not to move upward in the state in which the engagement protrusions 1610 are inserted into the engagement grooves 121. Since the support slider 1600 is disposed on the button member 700, the button member 700 and the support slider 1600 may be configured to be engaged not to move upward in the state in which the engagement protrusions 1610 are inserted into the engagement grooves 121.

The support slider 1600 may include at least one contact portion 1630 that comes into contact with the slide protrusion 516 in the release direction G2. The contact portion 1630 may have a contact surface corresponding to the shape of the outer surface of the slide protrusion 516 in the release direction G2. The contact surface of the contact portion 1630 is movable in the upper-lower direction along the slide protrusion 516 in the state of being in contact with the slide protrusion 516.

The support slider 1600 may include a connection extension portion 1620 extending while connecting the engagement protrusion 1610 and the corresponding contact portion 1630 to each other. In an embodiment, the connection extension portion 1620 may be configured to be compressively and elastically deformable in the engagement direction G1. Accordingly, even in the state in which the support slider 1600 is not lowered to a predetermined position and the engagement protrusion 1610 is not inserted into the engagement groove 121, the connection extension portion 1620 is compressively and elastically deformable in the engagement direction G1, and the contact portion 1630 is capable of coming into contact with a first portion 516a of the support slider 1600. For example, the connection extension portion 1620 may have a curved shape to be elastically deformable.

The support slider 1600 may include a spring engagement portion 1650 that protrudes to be engaged with the slider spring 1700. The spring engagement portion 1650 may be formed at a radially outward direction of the contact portion 1630. The slider spring 1700 is engaged with the spring engagement portion 1650.

In the present embodiment, the support slider includes a plurality of engagement operation portions 1600a, 1600b, and 1600c extending in respective engagement directions G1 around the slide protrusion 516 of the reservoir pushing member 500. In another embodiment (not illustrated), the support slider may have only one engagement operation portion.

Each of the plurality of engagement operation portions 1600a, 1600b, and 1600c may include the engagement protrusion 1610, the contact portion 1630, and the connection extension portion 1620. Each of the plurality of engaging operation portions 1600a, 1600b, and 1600c may include the spring engagement portion 1650. The plurality of engaging operation portions 1600a, 1600b, and 1600c may be arranged in the circumferential direction.

The support slider 1600 may include an elastic connection portion 1640 extending while connecting any two adjacent engagement operation portions to each other among the plurality of engagement operation portions 1600a, 1600b, and 1600c. The elastic connection portion 1640 may extend while connecting two adjacent contact portions 1630 to each other. The elastic connection portion 1640 may be configured to be elastically deformable. Specifically, when a plurality of contact portions 1630 of the plurality of engagement operation portions 1600a, 1600b, and 1600c move in the radially inward direction, the elastic connection portion 1640 may be contracted in the circumferential direction such that the two adjacent contact portions 1630 come closer to each other in the circumferential direction. In addition, when the plurality of contact portions 1630 of the plurality of engagement operation portions 1600a, 1600b, and 1600c move in the radially outward direction, the elastic connection portion 1640 may be stretched in the circumferential direction such that the two adjacent contact portions 1630 move away from each other in the circumferential direction. For example, the connection extension portion 1620 may have a curved shape to be elastically deformable.

The slider spring 1700 may press the contact portions 1630 in the release direction G2. By this, the contact portions 1630 may maintain the state of being in contact with the slide protrusion 516. The slider spring 1700 may be engaged with the spring engagement portion 1650 of the support slider 1600. The slider spring 1700 may be disposed in a radially outward direction of the contact portions 1630.

The slider spring 1700 may be formed of, for example, a metal material. The slider spring 1700 may be elastically deformed in the radially outward direction to apply an elastic restoring force to the contact portions 1630 in the radially inward direction. In the present embodiment, the slider spring 1700 is a C-shaped spring, but in another embodiment (not illustrated), one of various known manners for exerting an elastic force may be used. For example, the slider spring 1700 may include various types of members such as a compression spring, a tension spring, a torque spring, and an air spring, or may include a member made of a material such as rubber to be elastically compressed.

The support slider 1600 and the slider spring 1700 may be disposed inside the button member 700. The button member 700 may include the lower part 700B configured to press the upper end of the pressing member 1000. The button member 700 may include the upper part 700A coupled to the upper side of the lower part 700B.

The lower part 700B may be disposed below the support slider. The support slider 1600 may be disposed below the upper part 700A of the button member 700. The support slider 1600 may be disposed between the upper part 700A and the lower part 700B.

A hole 700h into which the slide protrusion 516 of the reservoir pushing member 500 is inserted may be formed at the lower part 700B. The hole 700h may penetrate the center of the lower part 700B in the upper-lower direction. A portion of the slide protrusion 516 that has passed to the upper side of the lower part 700B through the hole 700h may come into contact with the contact portions 1630 of the support slider 1600.

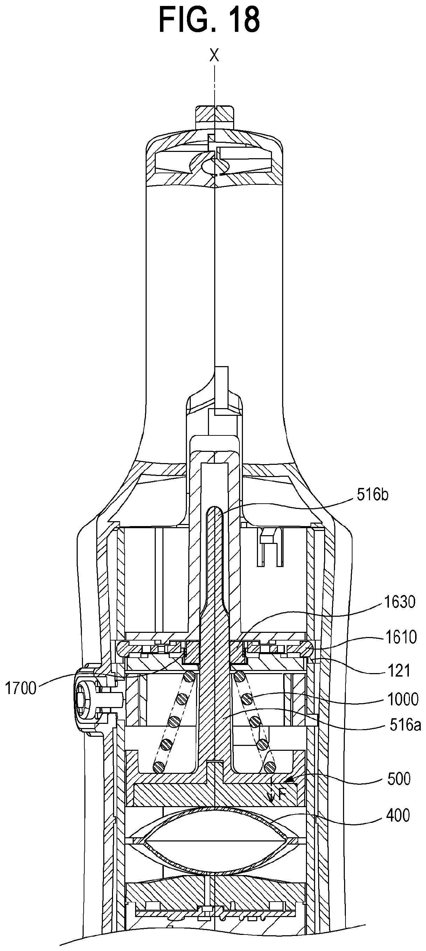

The pressing member 1000 may be configured to push the reservoir pushing member 500 downward while being stretched in the upper-lower direction to be elastically restored in the state in which the engagement protrusions 1610 are inserted into the engagement grooves 121. By presetting the elastic modulus of the pressing member 1000 to preset the elastic restoring force (*see* arrow F in FIGS. 18 and 19) of the pressing member 1000 that pushes the reservoir pushing member 500, it is possible to preset a rate of discharging the medicinal liquid from the inside of the reservoir 400.

In an embodiment, the reservoir pushing member 500 may be configured to be moved downward by being pushed downward by the pressing member 1000 without coming into contact with the button member 700. The pushing surface 512 of the reservoir pushing member 500 may be pushed by the pressing member 1000, rather than by the button member 700.

The slide protrusion 516 may include the first portion 516a constituting the lower portion of the slide protrusion 516 and a second portion 516b constituting the upper portion of the slide protrusion 516. The first portion 516a has a contact surface that is capable of contacting the contact portion 1630. The second portion 516b has a contact surface that is capable of contacting the contact portion 1630.

The contact surface of the first portion 516a is located on the side of the engagement direction G1 with reference to the contact surface of the second portion 516b (*see* FIGS. 17 and 21). In the present embodiment, the position of the circumferential surface of the first portion 516a is located in the radially outward direction from the position of the circumferential surface of the second portion 516b. For example, the circumferential length of the first portion 516a (*see* FIG. 21) may be greater than the circumferential length of the second portion 516b (*see* FIG. 22).

Hereinafter, the process of operating the button member 700, the support slider 1600, the pressing member 1000, the reservoir pushing member 500, the reservoir 400, and the like will be described with reference to FIGS. 16 to 22.

Referring to FIG. 16, in the initial state in which the medicinal liquid is stored in the reservoir 400 so that the reservoir is inflated, the button member 700 is in the state in which it is moved upward.

Referring to FIGS. 17 and 21, when the button member 700 moves in the downward direction MB, the support slider 1600 and the button member 700 integrally move downward. At this time, the support slider 1600 slides in the downward direction in the state of being in contact with the slide protrusion 516, and the pressing member 1000 is elastically compressed. In addition, when the support slider 1600 moves from the second portion 516b to the first portion 516a of the slide protrusion 516, the contact portions 1630 move in the engagement direction G1 and the slider spring 1700 is elastically deformed. When the contact portions 1630 of the support slider 1600 are in contact with the first portion 516a of the slide protrusion 516 before the support slider 1600 is inserted into and engaged with the engagement grooves 121, the connection extension portions 1620 of the support slider 1600 are elastically compressed in the engagement direction G1, and accordingly, the engagement protrusions 1610 press the inner surface of the guide part 100C. In addition, the elastic connection portions 1640 are also elastically deformable. Here, when the support slider 1600 is further lowered to reach the same level as the engagement grooves 121, the engagement protrusions 1610 are inserted into the engagement grooves 121 and engaged with the engagement grooves 121 while the connection extension portions 1620 are elastically restored. In the state in which the contact portions 1630 are in contact with the first portion 516a, the engagement protrusions 1610 are maintained in the state of being engaged with the engagement grooves 121, and the support slider 1600 and the button member 700 are maintained in the state of being fixed in the upper-lower direction with respect to case 100.

Referring to FIG. 18, since the pressing member 1000 is elastically compressed, the pressing member is elastically restored and presses the reservoir pushing member 500 downward. Accordingly, the reservoir pushing member 500 moves downward to press the reservoir 400, and the medicinal liquid inside the reservoir 400 is discharged.

Referring to FIGS. 19 and 22, when the reservoir pushing member 500 moves further downward to reach a predetermined position, the contact portions 1630 of the support slider 1600 come into contact with the second portion 516b of the slide protrusion 516. At this time, the slider spring 1700 is elastically restored, and the contact portions 1630 are moved in the radially inward direction to come into contact with the second portion 516b. Here, the elastic connection portions 1640 may also be elastically restored and the contact portions 1630 may come into contact with the second portion 516b. The predetermined position of the reservoir pushing member 500 may be set to a position in which the reservoir pushing member 500 fully compresses the reservoir 400. When the contact portions 1630 come into contact with the second portion 516b of the slide protrusion 516, the shape of the support slider 1600 is restored to the initial state of FIG. 16 so that the engagement protrusions 1610 come out of the engagement grooves 121.

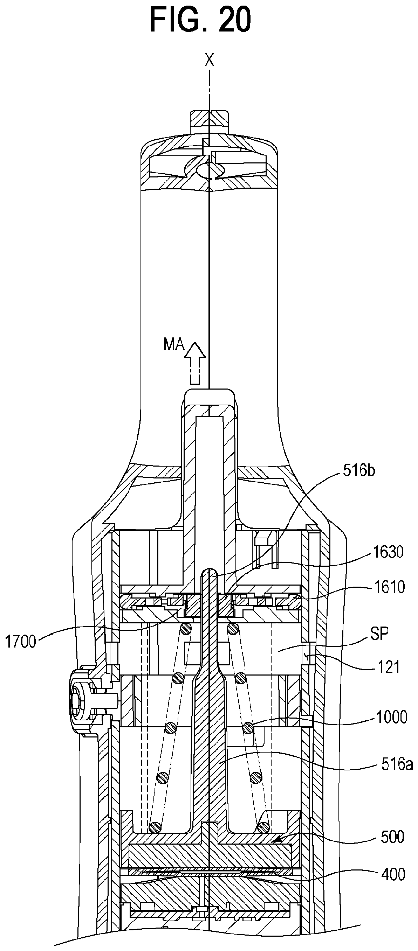

Referring to FIG. 20, when the engagement protrusions 1610 come out of the engagement grooves 121, the support slider 1600 and the button member 700 are integrally moved upward (MA) by the elastic restoring force of the pressing member 1000. At this time, the reservoir pushing member 500 and the reservoir support part 240 may maintain the state of being located close to each other. Thereafter, when the medicinal liquid is introduced into the reservoir 400, the reservoir pushing member 500 moves upward by the reservoir 400.

In another embodiment, the medicinal liquid control apparatus 80 may further include an additional elastic member SP that applies a restoring force to move the button member 700 upward (*see* FIG. 20). The elastic member SP may be configured to press the button member 700 such that the button member moves upward. The elastic member SP may be disposed between the button member 700 and the reservoir pushing member 500. The elastic member SP may be configured to have a longer elastic deformation length and a smaller elastic modulus compared to the pressing member 1000. The elastic member SP may use one of various known manners of exerting an elastic force. For example, the elastic member SP may include various types of members such as a compression spring, a torque spring, and an air spring, or may include a member made of a material such as rubber to be elastically compressed.

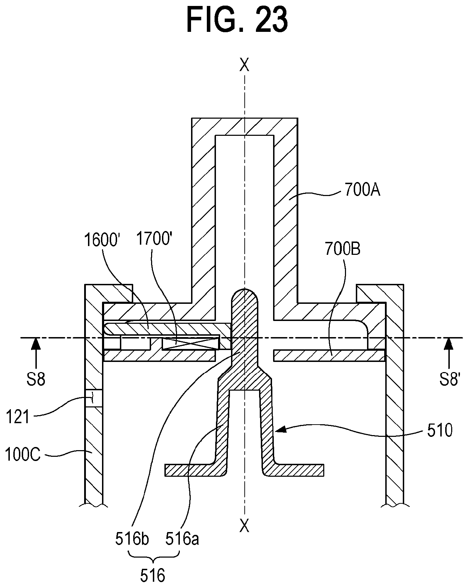

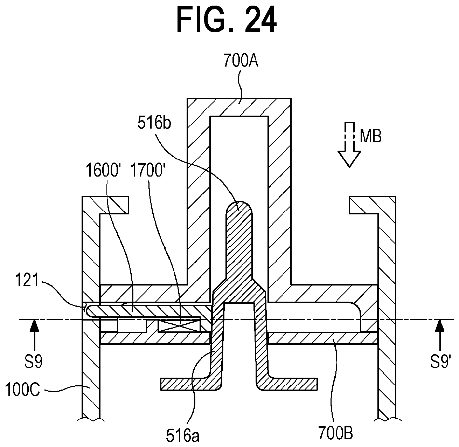

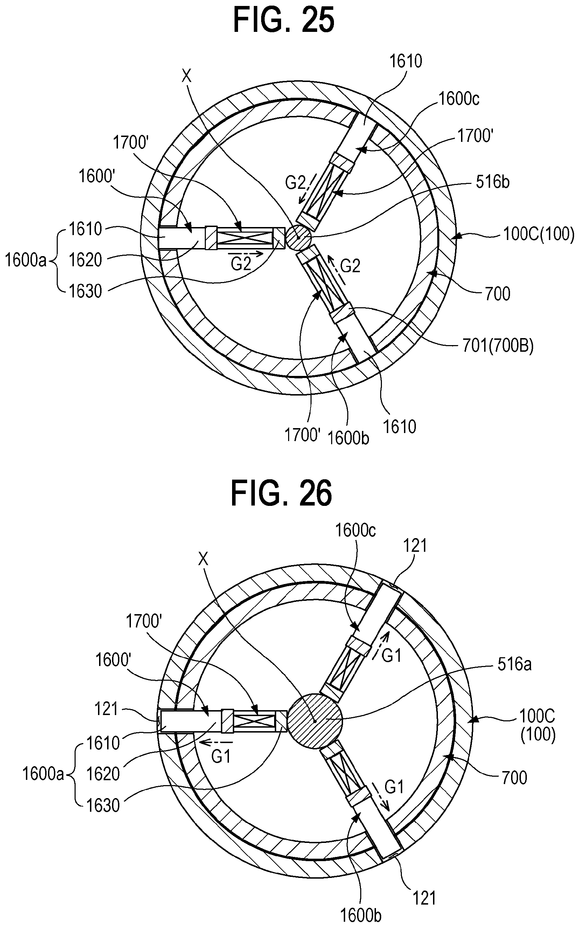

FIGS. 23 and 24 are partial longitudinal cross-sectional conceptual views of a medicinal liquid control apparatus including the support sliders 1600' and the slider springs 1700' according to another embodiment, in which FIG. 23 illustrates a state before the button member 700 is lowered and FIG. 24 illustrates a state in which the button member 700 is lowered. FIG. 25 is a cross-sectional conceptual view taken along line S8-S8' in FIG. 23. FIG. 26 is a cross-sectional conceptual view taken along line S9-S9' in FIG. 24.

Referring to FIGS. 23 to 26, a modification of the medicinal liquid control apparatus 80 will be described with reference to FIGS. 23 to 26. In the description of the medicinal liquid control apparatus according to the modification, the description of contents overlapping the foregoing contents will be omitted and the same reference numerals as the foregoing contents will denoted in the drawings. In the medicinal liquid control apparatus according to the modification, one ends of the slider springs 1700' may be configured to be supported by spring supports 701 formed at the button member 700, and the other ends of the slider springs 1700' may be supported on the support sliders 1600'. The slider springs 1700' may be configured to be elastically compressible in the engagement direction G1, and in the state in which the slider springs 1700' are elastically compressed, the support sliders 1600' may be pressed in the release direction G2. The plurality of engagement operation portions 1600a, 1600b, and 1600c may be formed separately from each other, but in another embodiment, the elastic connection portions 1640 may be connected to each other by the elastic connection portions 1640.

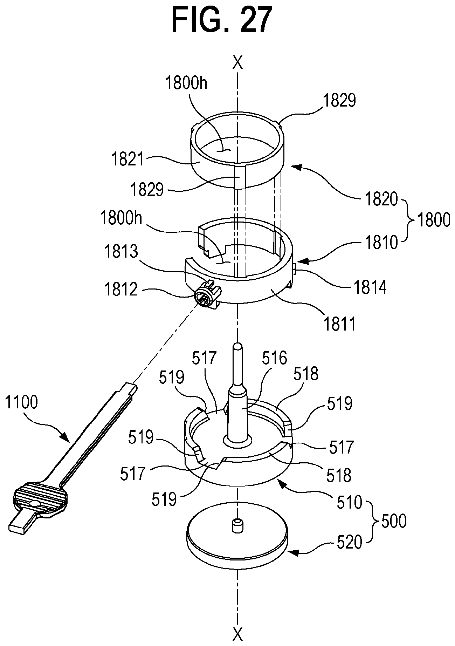

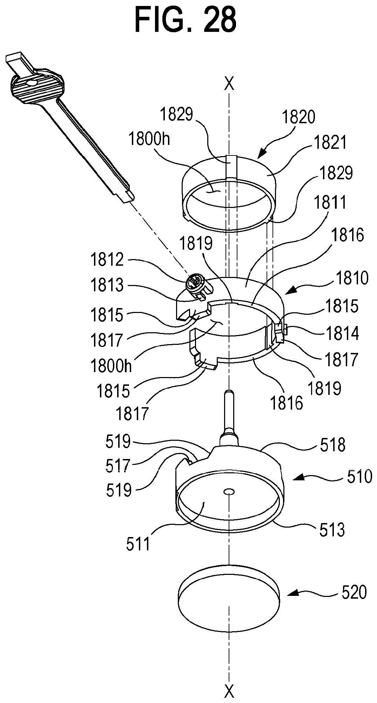

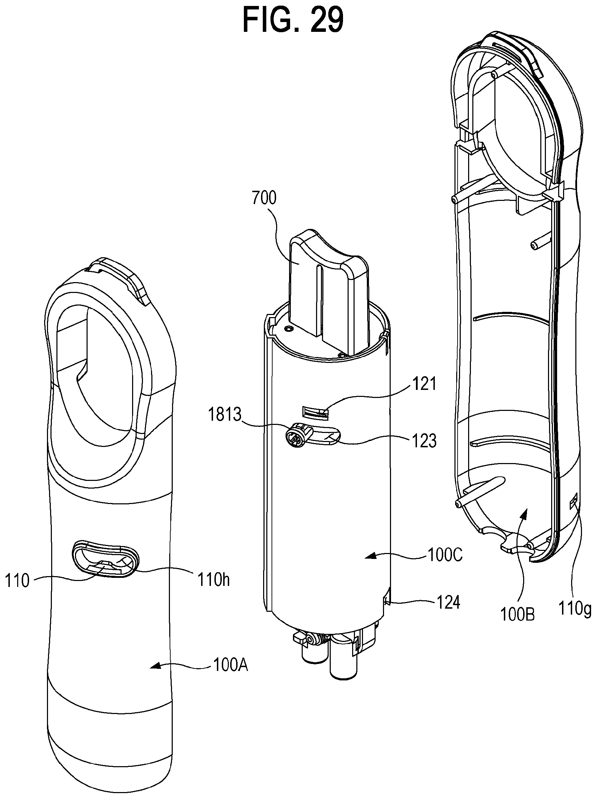

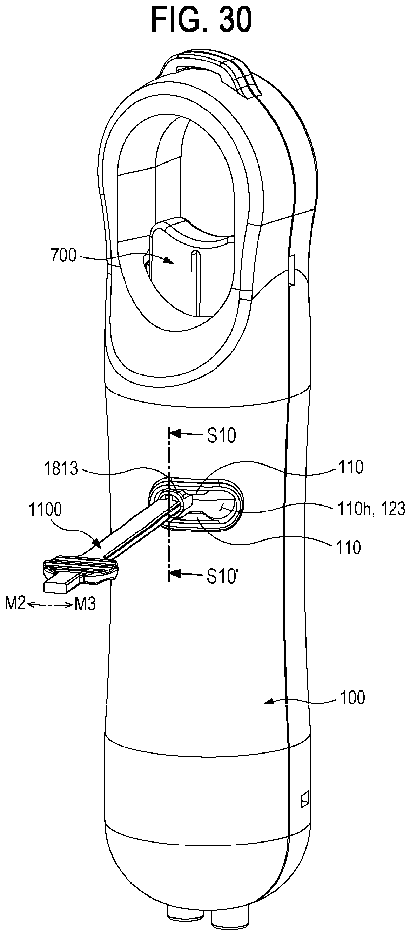

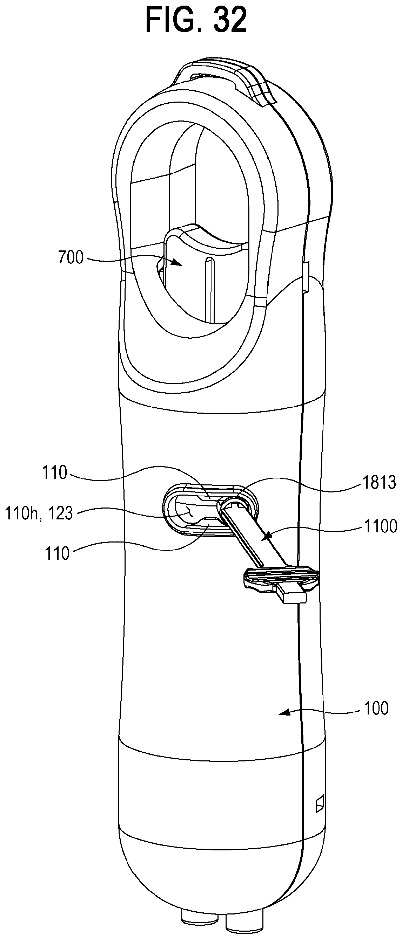

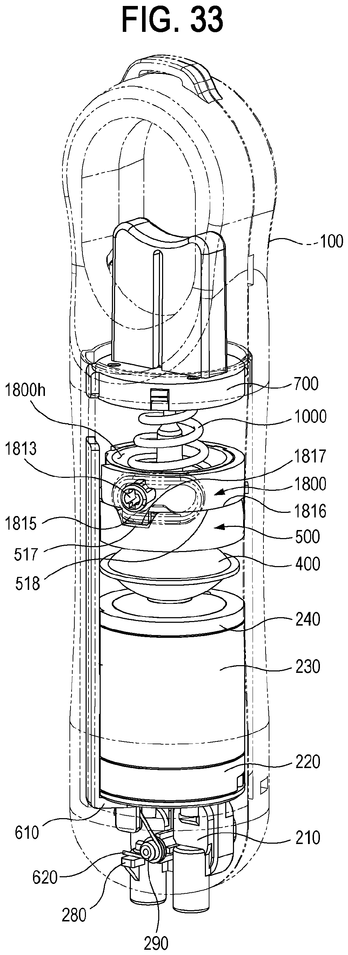

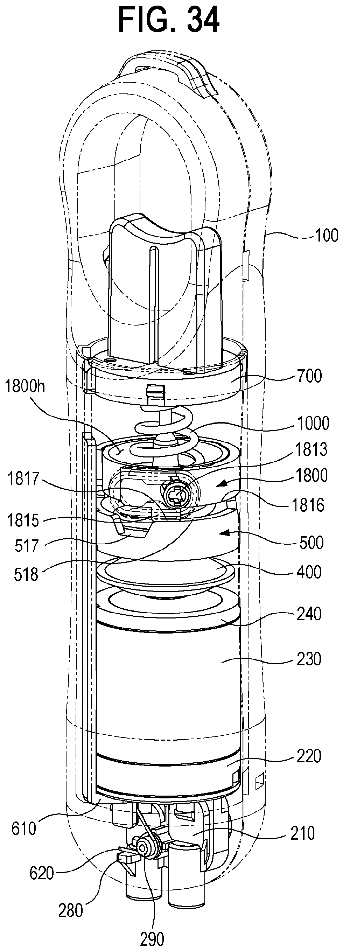

FIGS. 27 and 28 are exploded perspective views of the reservoir pushing member 500, the selector 1800, and the cotter 1100 of FIG. 6. FIG. 29 is a perspective view illustrating a state in which the case 100 is disassembled in the medicinal liquid control apparatus 80 of FIG. 3. FIGS. 30 to 32 are views illustrating states in which the selector 1800 is operated by using the cotter 1100 in the medicinal liquid control apparatus 80 of FIG. 3. FIG. 33 is a perspective view illustrating a state in which the case 100 is transparently processed in the medicinal liquid control apparatus 80 of FIG. 30. FIG. 34 is a perspective view illustrating a state in which the case 100 is transparently processed in the medicinal liquid control apparatus 80 of FIG. 32.

Referring to FIGS. 27 to 34, the medicinal liquid control apparatus 80 may be configured to change the maximum volume of the internal space 400s of the reservoir 400. A user such as a doctor may change the maximum volume of the internal space 400s by coupling the cotter 1100b to the selector operation portion 1812 and moving the position of the selector 1800 (*see* arrows M2 and M3).

In an embodiment, the medicinal liquid control apparatus 80 may include the reservoir 400, the reservoir pushing member 500, and the selector 1800. The medicinal liquid control apparatus 80 may further include the pressing member 1000 configured to be capable of pressing the reservoir pushing member 500 downward. The medicinal liquid control apparatus 80 may further include the cotter 1100 configured to be coupled to the selector 1800 to operate the selector 1800. The medicinal liquid control apparatus 80 may further include a case 100 surrounding the circumference of the selector 1800. The reservoir 400 may be configured to increase in length in the upper-lower direction when the volume of the internal space 400s increases. The reservoir pushing member 500 may be configured to be pushed upward by the reservoir 400 when the length in the upper-lower direction of the reservoir 400 increases. The reservoir pushing member 500 may be configured to be capable of pressing the reservoir 400 downward. The selector 1800 may be configured to be capable of contacting the reservoir pushing member 500 to limit the upward maximum movement position of the reservoir pushing member 500. The reservoir pushing member 500 may come into contact with the lower surface of the selector 1800 at the set maximum movement position.

The selector 1800 may be configured to be switchable from one of a plurality of set states, in which the maximum upward movement positions are set to be different from each other, to another one. The selector 1800 may be configured to be switchable from a first set state in which the maximum upward movement position is set to a first limit position to a second set state in which the maximum upward movement is set to a second limit position lower than the first limit position (*see* FIGS. 32 and 34). In the present embodiment, the selector 1800 is configured to be reversibly switchable from the first set state to the second set state, but in another embodiment (not illustrated), may be configured to be irreversibly switched from the first set state to the second set state. In the first set state illustrated with reference to FIGS. 30 and 33, a lower end surface 1817 of a limiter 1815 of the selector 1800 is in contact with a first level surface 517. At this time, the maximum upward movement position of the reservoir pushing member 500 is set to be relatively high. In the second set state illustrated with reference to FIGS. 32 and 34, the lower end surface of the limiter 1815 of the selector 1800 is in contact with a second level surface 518. At this time, the maximum upward movement position of the reservoir pushing member 500 is set to be relatively low.

In an embodiment, the selector 1800 moves (e.g., rotationally moves) in one direction M2 to be in the first set state located at the first position *(see* FIGS. 30 and 33), and the selector 1800 moves (e.g., rotationally moves) in the other direction M3 to be in the second set state located at the second position *(see* FIGS. 32 and 34). In the present embodiment, the first position and the second position differ from each other depending on the rotational movement about the central axis (X) of the selector 1800, but in another embodiment, the first position and the second position may differ from each other depending on the linear movement of the selector 1800 or the like.

Referring to FIGS. 27 and 28, in the present embodiment, the selector 1800 includes a first selector part 1810 and a second selector part 1820 coupled to each other, but in another embodiment (not illustrated), the selector 1800 may be an integrally configured component. The first selector part 1810 may extend along an inner circumferential surface of the case 100. One end and the other end of the first selector part 1810 in the circumferential direction (circumferential direction) may be spaced apart from each other. The second selector part 1820 may be coupled to the inner circumferential surface of the first selector part. The second selector part 1820 may be formed in a ring shape extending in the circumferential direction (circumferential direction). By this, while it is convenient to elastically deform and insert the first selector part 1810 into the inside of the case 100 during a manufacturing process, it is possible to reinforce the rigidity of the selector 1800 by using the second selector part 1820.

The first selector part 1810 may include a selector body 1811 and the selector operation portion 1812. The first selector part 1810 may include an engagement portion 1813. The first selector part 1810 may include a guide protrusion 1814. The first selector part 1810 may include the limiter 1815.

The second selector part 1820 may include a reinforcing body 1821 formed in a ring shape. The second selector part 1820 may include a coupling portion 1829 protruding radially outward from the reinforcing body 1821. A plurality of coupling portions 1829 may be disposed to be spaced apart from each other in the circumferential direction. The radially inward surface of the first selector part 1810 may include a coupling counterpart 1819 to which the coupling portion 1829 is engaged and coupled.

An opening 1800h penetrating the selector in the upper-lower direction may be formed at the selector 1800. The pressing member 1000 may pass through the opening downward to press the reservoir pushing member 500 downward. As described above, in the present embodiment, the pressing member 1000 may be configured as a spring elastically and compressively deformed, or as a bar or the like in other embodiments.

Referring to FIGS. 27 to 34, the selector 1800 may be configured to be switched from the first set state to the second set state by rotating in the circumferential direction thereof. The selector 1800 may be configured to be fixed in the position in the upper-lower direction with respect to the guide part 100C and to be rotatable with respect to the guide part 100C. The selector 1800 may rotate about the central axis X.

The reservoir pushing member 500 and the selector 1800 are configured to be capable of contacting each other vertically. The selector 1800 may be configured to be switchable from the first set state to the second set state when a second contact surface which is capable of contacting a first contact surface varies, wherein the first contact surface is located on one of the selector 1800 and the reservoir pushing member 500 and the second contact surface is located on the other one of the selector and the reservoir pushing member. The first contact surface and the second contact surface may be configured to be capable of contacting each other in the upper-lower direction. For example, the first contact surface is a second lower end surface 1817 of the selector 1800, the second contact surface in the first set state is the first level surface 517, and the second contact surface in the second set state is the second level surface 518. As another example, the first contact surface is the second level surface 518 of the reservoir pushing member 500, the second contact surface in the first set state is a first lower end surface 1816, and the second contact surface in the second set state is the second lower end surface 1817.

The second contact surface in the second set state is located in the direction facing the first contact surface with reference to the second contact surface in the first set state. For example, in the reservoir pushing member 500, the second level surface 518 as the second contact surface in the second set state is located in the upper direction with reference to the first level surface 517 as the second contact surface in the first set state. As another example, in the selector 1800, the second lower end surface 1817 as the second contact surface in the second set state is located in the lower direction with reference to the first lower end surface 1816 as the second contact surface in the first set state.

Of the selector 1800 and the reservoir pushing member 500, one having the first contact surface protrudes toward the other one having the second contact surface to provide the first contact surface facing the other one. For example, the selector 1800 protrudes toward the reservoir pushing member 500 to provide the second lower end surface 1817 facing the reservoir pushing member 500. As another example, the reservoir pushing member 500 protrudes toward the selector 1800 to provide the second level surface 518 facing the selector 1800.

In the present embodiment, the second contact surface is configured at two different levels. However, in another embodiment (not illustrated), the second contact surface may be configured at three or more different levels, and in this case, the maximum volume of the internal space 400s of the reservoir 400 may be changed over three or more levels.

In the present embodiment, in the first set state, the first lower end surface 1816 and the second lower end surface 1817 come into contact with the upper surfaces 518 and 517 of the reservoir pushing member 500, respectively. However, in another embodiment (not illustrated), in the first set state, only one of the first lower end surface 1816 and the second lower end surface 1817 is capable of coming into contact with the upper surfaces 518 and 517 of the reservoir pushing member 500.

One of the selector 1800 and the reservoir pushing member 500 may include at least one limiter 1815 protruding toward the other one, and the other one of the selector 1800 and the reservoir pushing member 500 may include the first level surface 517 and the second level surface 518 with which the limiter 1815 is capable of coming in contact. The first level surface 517 and the second level surface 518 may have different levels in the upper-lower direction. In the present embodiment, the selector 1800 includes the limiter 1815 protruding downward, and the reservoir pushing member 500 includes the first level surface 517 and the second level surface 518. However, in another aspect, it may be understood that the reservoir pushing member 500 includes a limiter 518 protruding upward and the selector 1800 includes a first level surface 1816 and a second level surface 1817.

The selector 1800 may include the at least one limiter 1815 protruding downward. The at least one limiter 1815 may include a plurality of limiters 1815 arranged to be spaced apart from each other along the circumferential direction of the selector 1800. By this, it is possible to more stably limit the maximum upward movement position of the reservoir pushing member 500 and to easily switch the set state by positioning the first level surface 1816 and the second level surface 1817 on the rotational movement track of the limiters 1815 while enabling the stable rotation movement of the selector 1800.

The selector 1800 may include the second lower end surface 1817 forming the lower end of the limiter 1815, and the first lower end surface 1816 having a higher level than the second lower end surface 1817. The first lower end surface 1816 and the second lower end surface 1817 may be arranged in the rotational movement direction of the selector 1800.

The reservoir pushing member 500 may include the second level surface 518 providing the upper end, and the first level surface 517 having a lower level than the second level surface 518. The first level surface 517 and the second level surface 518 may be arranged in the rotational movement direction of the selector 1800. The reservoir pushing member 500 may include a plurality of first level surfaces 517 corresponding to the plurality of limiters 1815. The reservoir pushing member 500 may include a plurality of second level surfaces 518 corresponding to the plurality of limiters 1815.

The reservoir pushing member 500 may include inclined surfaces 519 interconnecting the first level surfaces 517 and the second level surfaces 518. The inclined surfaces 519 may extend to be inclined along the rotational movement direction of the selector 1800.

The selector 1800 may include the selector body 1811 extending in the circumferential direction of the selector 1800. The limiters 1815 may protrude downward from the selector body 1811.

The selector 1800 may include a guide protrusion 1814 protruding radially outward from the selector body 1811. The guide protrusion 1814 may be inserted into a selector guide groove 122 formed at the guide part 100C (*see* FIG. 4). The guide protrusion 1814 may move in the circumferential direction along the selector guide groove 122.

The selector 1800 may include the selector operation portion 1812 penetrating the case 100 from the inside to the outside. The selector operation portion 1812 may protrude to the outside of the case 100. The selector hole 110h, 123 through which the selector operation portion 1812 passes is formed at the case 100 (*see* FIG. 29). The selector operation portion 1812 may be configured to be disposed at a first set position in the selector hole 110h, 123 in the first set state (*see* FIG. 30) and disposed at a second set position different from the first set position in the selector hole 110h, 123 in the second set state (*see* FIG. 32).

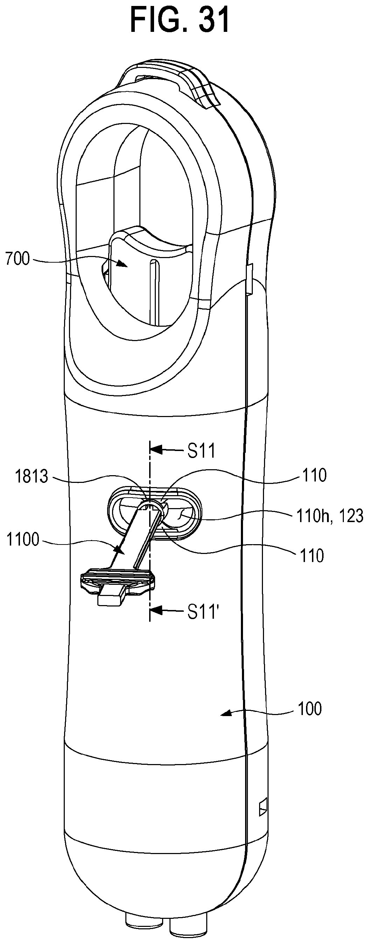

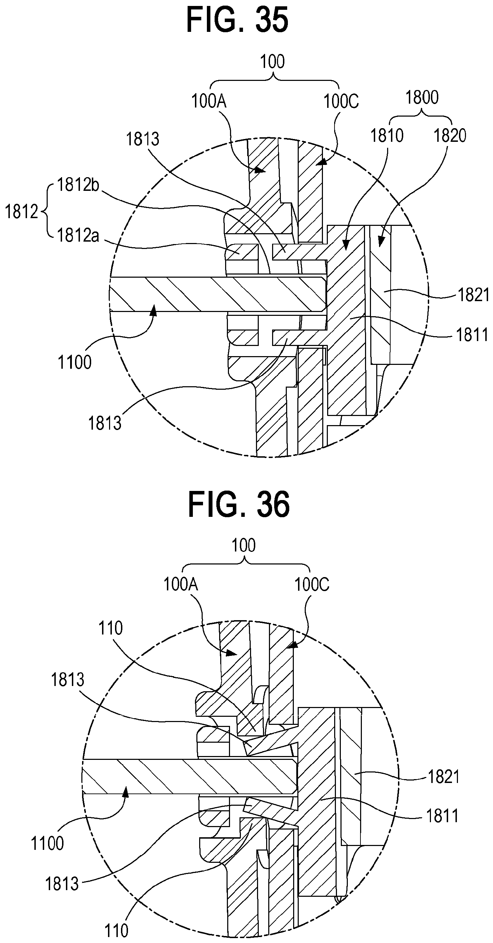

FIG. 35 is a cross-sectional view of the medicinal liquid control apparatus 80 taken along line S10-S10' in FIG. 30. FIG. 36 is a cross-sectional view of the medicinal liquid control apparatus 80 taken along line S11-S11' in FIG. 31.

Referring to FIG. 35, the selector operation portion 1812 may include a head 1812a at which a groove in a portion exposed to the outside of the case 100 is formed. The groove may be formed by being recessed in the radially inward direction in the head 1812a. The head 1812a may be configured to be coupled with the cotter 1100. The cotter 1100 may be configured to be insertable into the groove of the head 1812a.

The selector operation portion 1812 may include a connection portion 1812b extending to interconnect the head 1812a and the selector body 1811. The connection portion 1812b may extend in the radially outward direction from the selector body 1811. The connection portion 1812b may pass through the selector hole 110h, 123.

Referring to FIGS. 31, 35, and 36, one of the case 100 and the selector 1800 may include the engagement portion 1813 that is elastically deformable by the other one. The engagement portion 1813 may be configured to be elastically deformable when the selector operation portion 1812 moves from "the first set portion or the second set position" in the selector hole 110h, 123 (e.g., the state of FIG. 30 or FIG. 32) to "an intermediate position between the first set position and the second set position" (e.g., the state of FIG. 31). The other one of the case 100 and the selector 1800 may include a protrusion 110 that comes into contact with and presses the engagement portion 1813 when the selector operation portion 1812 is located at "the intermediate position between the first and second set positions." The engagement portion 1813 may be elastically deformed by the pressing of the protrusion 110, and the elastic restoring force of the engagement portion 1813 may cause the selector operation portion 1812 to go off the movement path between the first position and the second position so that the selector operation portion 1812 may be guided to be disposed at a set position that has been set in advance. In the present embodiment, the selector 1800 includes the engagement portion 1813 and the case 100 includes the protrusion 110, but in an embodiment (not illustrated), the case 100 may include the engagement portion and the selector 1800 may include the protrusion.

This may make it difficult for the selector 1800 to maintain the position between the first position and the second position by causing the engagement portion 1813 to be elastically deformed in the process in which the selector 1800 moves (e.g., rotationally moves) between the first position and the second position.

In the present embodiment, the engagement portion 1813 may protrude from the selector body 1811 in a radially upward direction. The engagement portion 1813 may provide a free end in the radially outward direction. However, in another embodiment (not illustrated), the engagement portion may protrude in the radially inward direction from the head 1812a.

The engagement portion 1813 may be arranged side by side with the connection portion 1812b. The engagement portion 1813 may be disposed to be vertically spaced apart from the connection portion 1812b. The engagement portion 1813 may be disposed in the selector hole 110h, 123. A pair of engagement portions 1813 may be provided with the connection portion 1812b interposed between the pair of engagement portions 1813. The engagement portions 1813 may be configured to be elastically deformable while being bent in a direction in which the connection portion 1812b is disposed.

The case 100 may include the protrusion 110 protruding to narrow the middle point of the selector hole 110h, 123. The protrusion 110 of the case 100 may protrude in a direction transverse to the movement path of the selector operation portion 1812. The protrusion 110 may protrude to have a portion of the selector hole 110h, 123. A pair of protrusions 110 corresponding to the pair of engagement portions 1813 may be provided. The protruding end of the protrusion 110 may press the engagement portion 1813, so that the engagement portion 1813 may be elastically bent and deformed.

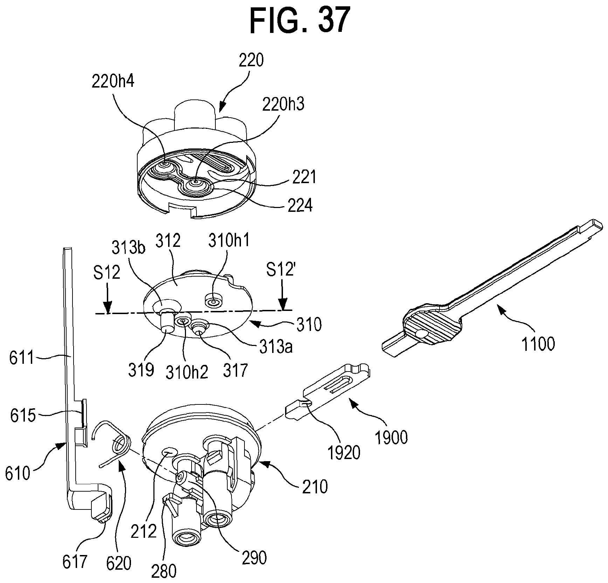

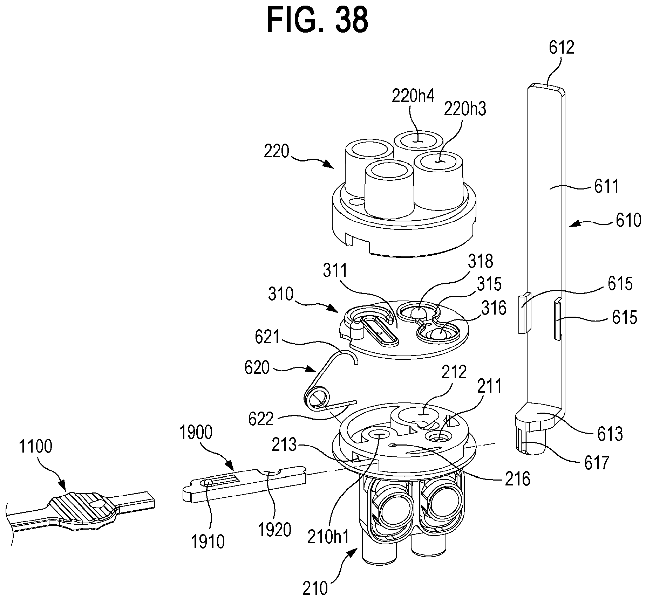

FIGS. 37 and 38 illustrate some components including the lock bar 610, the lock elastic member 620, the switching member 1900, and the like in the disassembled state.

Referring to FIGS. 37 to 44, the medicinal liquid control apparatus 80 according to an embodiment may include at least one channel valve configured to switch a partial section of the medicinal liquid channel from an open state to a closed state. In an embodiment, the medicinal liquid control apparatus 80 may include the channel forming part 220 and the valve plate 310. The medicinal liquid control apparatus 80 may further include the valve support part 210.

In the present embodiment, the at least one channel valve includes the first channel valve 316 and the second channel valve 318. This makes it possible to inject the medicinal liquid stored in the reservoir 400 of the medicinal liquid control apparatus 80 into a patient at a necessary time, and to control the injection dose of the medicinal liquid in various manners by stopping the injection of the medicinal liquid continuously supplied through the first channel P1.

In another embodiment, the at least one channel valve may include the second channel valve 318, but may not include the first channel valve 316. In another embodiment, in the medicinal liquid control apparatus 80 provided with the first channel P1 only without the second channel P2, the at least one channel valve may include the first channel valve 316, but may not include the second channel valve 318.

The second channel valve 318 may be configured to open/close the second channel P2. The first channel valve 316 may be configured to switch the section between the branching point Qd and the merging point Qu in the first channel P1 from an open state to a closed state. In the present embodiment, the first channel valve 316 is configured to prevent the section between the branching point Qd and the merging point Qu in the first channel P1 from being switched from the closed state to the open state, but in another embodiment, the first channel valve 316 may be configured to be capable of opening/closing the section between the branching point Qd and the junction point Qu in the first channel P1. That is, a medicinal liquid channel locking apparatus may be configured to change the first channel P1 from a closed state to an open state. The first channel valve 316 and the second channel valve 318 may be configured to operate independently of each other.

In an embodiment, the medicinal liquid control apparatus 80 may further include the selector 1800 while including the first and second channel valves 316 and 318. In this embodiment, the reservoir 400 may be configured to increase in length in a predetermined direction when the volume of the internal space 400s increases. The reservoir pushing member 500 may be configured to be pushed in a predetermined direction by the reservoir 400 when the length in the predetermined direction of the reservoir 400 increases. The selector 1800 may be configured to be capable of contacting the reservoir pushing member 500 to limit the maximum movement position of the reservoir pushing member 500 in the predetermined direction. The selector 1800 may be configured to enable an operation of selecting one of a plurality of set states in which the maximum movement positions in the predetermined direction are set to be different from each other. This makes it possible to control the injection dose of the medicinal liquid in more various manners by combining whether to open or close the second channel, the change in the storage amount of the medicinal liquid of the reservoir 400, and whether to open or close the section between the branching point Qd and the merging point Qu in the first channel P1. In the present embodiment, the predetermined direction may be an upward direction.

Referring to FIGS. 37 to 44, it can be understood that the following descriptions for the channel valves 316 and 318, the extension holes 220h3 and 220h4, the pushing portions 317 and 319, and inclined portions 313a and 313b are made based on one of the first channel valve 316 and the second channel valve 318.

The channel forming part 220 defines the extension holes 220h3 and 220h4. One ends (lower ends) of the extension holes 220h3 and 220h4 may be located in the lower surface of the channel forming part 220. The channel forming part 220 may define a first extension hole 220h3 constituting a portion of the first channel P1. The channel forming part 220 may define a second extension hole 220h4 constituting a portion of the second channel P2.

The valve plate 310 may cover at least a portion of the lower surface including the portion where the one ends of the extension holes 220h3 and 220h4 are located. The valve plate 310 may include the channel valves 316 and 318 protruding upward toward the one ends of the extension holes 220h3 and 220h4. The first channel valve 316 may protrude toward the one end of the first extension hole 220h3. The second channel valve 318 may protrude toward the one end of the second extension hole 220h4. The valve plate 310 may include pushing portions 317 and 319 located at the lower ends of the channel valves 316 and 318.

In an embodiment, the first channel valve 316 and the second channel valve 318 are formed at the valve plate 310. The valve plate 310 covers a portion of the channel forming part 220. The valve plate 310 may be configured to be elastically deformed or elastically restored so that the first channel valve 316 is operated. The valve plate 310 may be configured to be elastically deformed or elastically restored so that the second channel valve 318 is operated.

Merging guide channels P1e and P2e partitioned by the channel forming part 220 and the valve plate 310 may be defined between the channel forming part 220 and the valve plate 310. In the released state in which the upward pressing on a first pushing portion 317 is released, the extension hole 220h3 and the guide channel P1e are interconnected through the one end of the extension hole 220h3 to constitute a portion of the medicinal liquid channel. In the released state in which the upward pressing on a second pushing portion 319 is released, the extension hole 220h4 and the guide channel P2e are interconnected through the one end of the extension hole 220h4 to constitute a portion of the medicinal liquid channel.

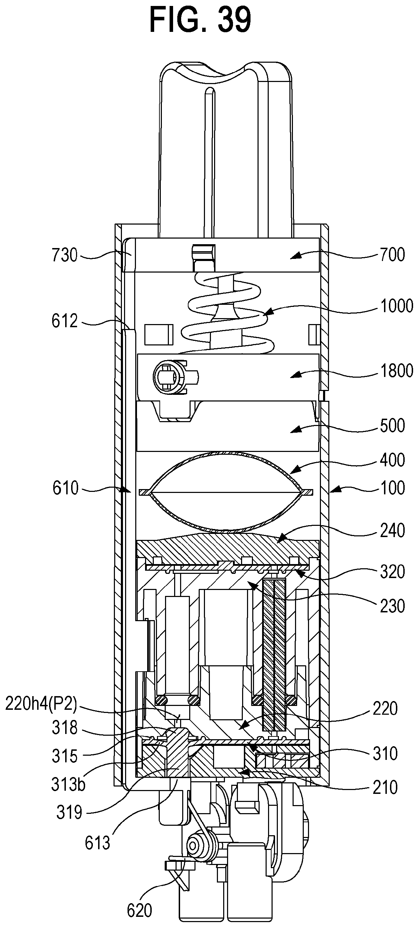

A gap may be formed between the channel forming part 220 and the valve plate 310 along the circumference of the second channel valve 318 *(see* FIGS. 39 to 41). In the released state in which the upward pressing on the second pushing portion 319 is released, the second extension hole 220h4 and the gap are interconnected through the one end of the second extension hole 220h4 to constitute a portion of the medicinal liquid channel.

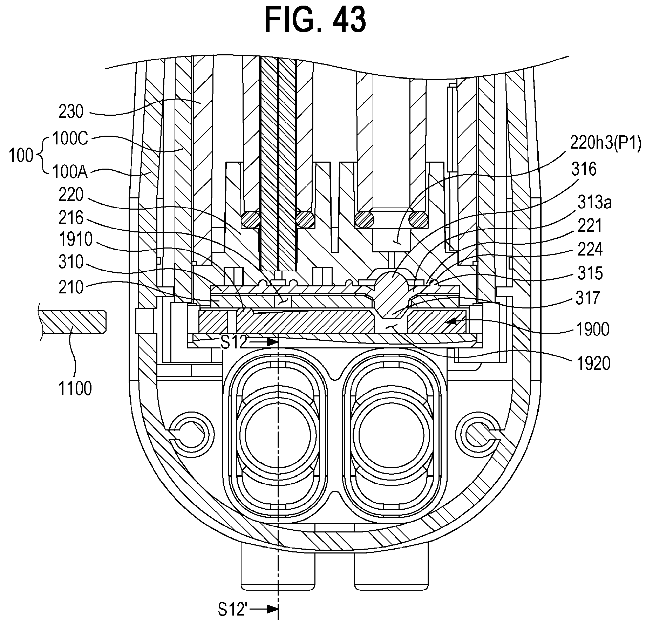

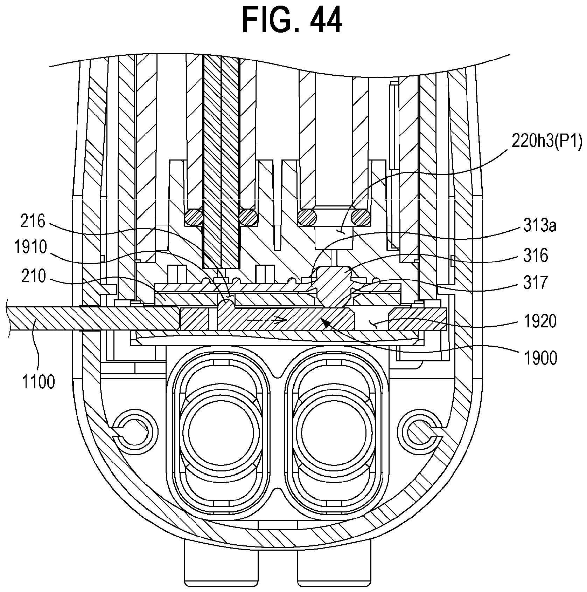

A gap may be formed between the channel forming part 220 and the valve plate 310 along the circumference of the first channel valve 316 *(see* FIGS. 43 and 44). In the released state in which the upward pressing on the first pushing portion 317 is released, the first extension hole 220h3 and the gap are interconnected through the one end of the first extension hole 220h3 to constitute a portion of the medicinal liquid channel.

The extension holes 220h3 and 220h4 may constitute a portion of the first channel P1 or a portion of the second channel P2 in the section between the branching point Qd and the merging point Qu in the medicinal liquid channel. For example, the first extension hole 220h3 may constitute a portion of the first channel P1 in the section between the branching point Qd and the merging point Qu in the medicinal liquid channel. As another example, the second extension hole 220h4 may constitute a portion of the second channel P2 in the section between the branching point Qd and the merging point Qu in the medicinal liquid channel.

In the locked state in which the pushing portions 317 and 319 are pressed upward, the valve plate 310 is elastically deformed so that the channel valves 316 and 318 block the one ends of the extension holes 220h3 and 220h4. In the released state in which the upward pressing on the pushing portions 317 and 319 is released, the valve plate 310 may be elastically restored such that the channel valves 316 and 318 open the one ends of the extension holes 220h3 and 220h4.

For example, in the locked state in which the first pushing portion 317 is pressed upward, the valve plate 310 may be elastically deformed such that the first channel valve 316 closes the one end of the first extension hole 220h3. In the released state in which the upward pressing on the first pushing portion 317 is released, the valve plate 310 may be elastically restored such that the first channel valve 316 opens the one end of the first extension hole 220h3.

As another example, in the locked state in which the second pushing portion 319 is pressed upward, the valve plate 310 may be elastically deformed such that the second channel valve 318 closes the one end of the second extension hole 220h4. In the released state in which the upward pressing on the second pushing portion 319 is released, the valve plate 310 may be elastically restored such that the second channel valve 318 opens the one end of the second extension hole 220h4.

In an embodiment, the valve plate 310 may include the first pushing portion 317 located at an end in a direction opposite to a protrusion direction of the first channel valve 316. The valve plate 310 may include the second pushing portion 319 located at an end in a direction opposite to a protrusion direction of the second channel valve 318. The first channel valve 316 may be configured to be operated when the first pushing portion 317 is pushed or released. The second channel valve 318 may be configured to be operated when the second pushing portion 319 is pushed or released.

The pushing portions 317 and 319 may protrude downward. The pushing portions 317 and 319 may protrude downward from the lower surface of the valve plate 310. The first pushing portion 317 may be located on a downward extension line of the first channel valve 316. The second pushing portion 319 may be located on a downward extension line of the second channel valve 318. The second pushing portion 319 may protrude downward to be longer than the first pushing portion 317.

Pushing portion penetration holes 211 and 212 through which the pushing portions 317 and 319 pass downward may be formed at the valve support part 210. The first pushing portion penetration hole 211 through which the first pushing portion 317 passes downward may be formed at the valve support part 210. The second pushing portion penetration hole 212 through which the second pushing portion 319 passes downward may be formed at the valve support part 210. By the pushing portion penetration holes 211 and 212, when the pushing portions 317 and 319 are pushed upward, a predetermined elastic deformation can be smoothly induced without buckling of the pushing portions 317 and 319.

The lower ends of the pushing portions 317 and 319 may be configured to be capable of being pressed upward. For example, the first pushing portion 317 may be pressed upward by the switching member 1900. As another example, the lower end of the second pushing portion 319 may be pressed upward by the lock bar 610.

The valve plate 310 may further include the inclined portions 313a and 313b extending obliquely upward from the circumferences of the pushing portions 317 and 319 in the released state. The inclined portions 313a and 313b may extend along the circumferences of the pushing portions 317 and 319. The inclined portions 313a and 313b may be inclined to protrude downward from the lower surface of the valve plate 310. The inclined portions 313a and 313b may be inclined so that the upper surface of the valve plate 310 is recessed downward. A first inclined portion 313a may be provided along the circumference of the first pushing portion 317. A second inclined portion 313b may be provided along the circumference of the second pushing portion 319. By the inclined portions 313a and 313b, the movable range of the channel valves 316 and 318 in the upper-lower direction according to elastic deformation can be increased, and a gap can be easily provided between the valve plate 310 and the channel forming part 220.

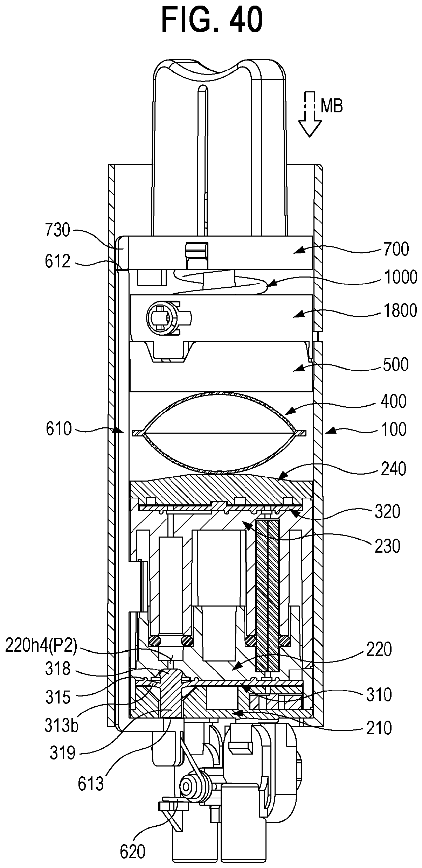

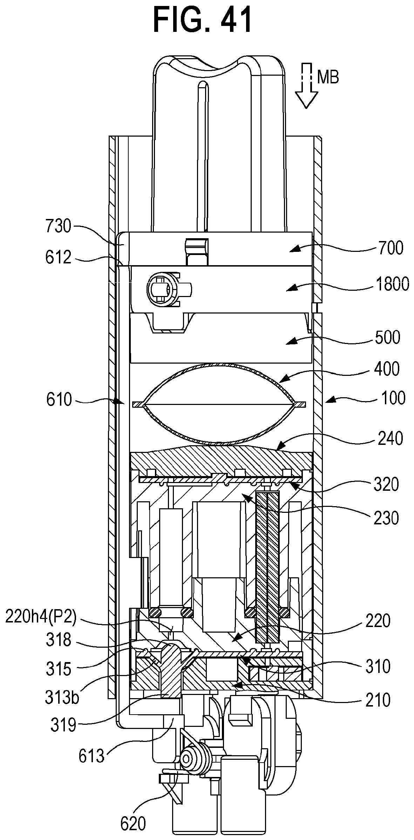

FIG. 39 is a cross-sectional view of some components of the medicinal liquid control apparatus 80 taken along line S12-S12' in FIG. 37, illustrating a state in which the channel P2 is blocked by the valve 318. FIG. 40 is a cross-sectional view illustrating a state in which the button member 700 is lowered by a partial section from FIG. 39 and the button member 700 and the lock bar 610 come into contact with each other. FIG. 41 is a cross-sectional view illustrating a state in which the button member 700 is further lowered from FIG. 40 to move the lock bar 610 so that the channel P2 is opened by the valve 318.

Hereinafter, a configuration related to the second channel valve 318 will be described with reference to FIGS. 37 to 41. The medicinal liquid control apparatus 80 may be configured to change the second channel P2 from the closed state to the open state when the user pushes the button member 700. In an embodiment, the medicinal liquid control apparatus 80 includes the lock bar 610 and the lock elastic member 620. The medicinal liquid control apparatus 80 may further include a button member. The medicinal liquid control apparatus 80 may further include the channel forming part 220 and the valve plate 310. The medicinal liquid control apparatus 80 may further include the valve support part 210.

One end 621 of the lock elastic member 620 may be supported by the lock bar 610. The body 200 may include a spring support portion 280 that supports the other end 622 of the lock elastic member 620. The body 200 may include a spring arrangement portion 290 on which the lock elastic member 620 is disposed *(see* FIG. 20).

The valve plate 310 may include the second pushing portion 319 that is pushed by the lock bar 610. The valve plate 310 may include the second channel valve 318 disposed and protruding on the side opposite to the second pushing portion 319.

The button member 700 may be configured to be movable in the upper-lower direction. The button member 700 may be configured to move downward to press the lock bar 610 downward when switched from the locked state in which the second pushing portion 319 is pressed upward to the released state. In the state in which the above-described support slider 1600 is engaged with the guide part 100C, the button member 700 may maintain the state of pushing the lock bar 610 downward while maintaining a constant position together with the support slider 1600. In addition, the button member 700 may be configured to be movable in a predetermined pushing direction such that the reservoir pushing member 500 presses the reservoir 400 and the second channel valve 318 opens the second channel P2. In the present embodiment, the predetermined pushing direction is a downward direction.

When the button member 700 is switched from the released state in which the upward pressing on the second pushing portion 319 is released to the locked state, the button member 700 may move upward to release the pressing on the lock bar 610. At this time, the button member 700 may be upwardly spaced apart from the lock bar 610. In addition, the button member 700 may be configured to be movable in a direction opposite to the predetermined pushing direction such that the reservoir pushing member 500 releases the pressing on the reservoir 400 and the second channel valve 318 closes the second channel P2.

The button member 700 may include the protrusion 730 protruding to one side to press the lock bar 610. The protrusion 730 may protrude radially outward from the button member 700. The protrusion 730 may press the upper end 612 of the lock bar 610.

The lock bar 610 may be configured to be movable in the upper-lower direction. The lock bar 610 may be configured to be movable in the upper-lower direction with respect to the case 100. The lock bar 610 may be configured to press the second pushing portion 319 upward by moving upward when switched from the released state in which the upward pressing on the second pushing portion 319 is released to the locked state. The lock bar 610 may be configured to release the upward pressing on the second pushing portion 319 by moving downward when switched from the locked state to the released state.

The lock bar 610 may include a lock pushing portion 612 configured to be pushed downward by the button member 700. The lock pushing portion 612 may form the upper end of the lock bar 610. The lock bar 610 may include a lock extension portion 611 extending from the lock pushing portion 612 to the lower side of the second pushing portion 319. The lock extension portion 611 may extend in the upper-lower direction. The lock bar 610 may include a lock pressing portion 613 configured to press the lower end of the second pushing portion 319 upward. The lock pressing portion 613 may extend radially inward from the lower end of the lock extension portion 611. The lock pressing portion 613 includes an upper surface that comes into contact with the lower end of the second pushing portion 319. The lock bar 610 may include a lock slider 615 guided by the body 200 to be guided in the moving direction in the upper-lower direction. The lock slider 615 may protrude radially inward from the lock extension portion 611 and extend in the upper-lower direction. The lock bar 610 may include a lock elastic member support portion 617 on which the one end 621 of the lock elastic member 620 is supported. The lock elastic member support portion 617 may form a groove into which the one end 621 of the lock elastic member 620 is inserted. The groove of the lock elastic member support portion 617 may be formed by being recessed upward. The lock elastic member support portion 617 may be disposed below the lock pressing portion 613.

Hereinafter, the process of operating the button member 700, the lock bar 610, the lock elastic member 620, the second channel valve 318, and the like will be described with reference to FIGS. 39 to 41.

Referring to FIG. 39, in the initial state in which the button member 700 is moved upward without being pushed, the lock bar 610 is in the state of being pushed upward by the elastic restoring force of the lock elastic member 620. Accordingly, the lock bar 610 pushes the second pushing portion 319 upward, and as the second pushing portion 319 is pushed, the valve plate 310 is elastically deformed, and the second channel valve 318 moves upward to close the channel P2. In the present embodiment, the button member 700 and the lock bar 610 are set to be spaced apart from each other in the initial state, but in another embodiment, the button member 700 and the lock bar 610 may be set to be in contact with each other in the initial state.

Referring to FIG. 40, the button member 700 may be moved downward by a certain distance to bring the button member 700 and the lock bar 610 into contact with each other. At this time, the lock pushing portion 612 of the lock bar 610 and the protrusion 730 of the button member 700 may come into contact with each other. During the change from the initial state to the contact state, there is no change in the position of the lock bar 610, and the second channel valve 318 maintains the closed state of the channel P2.

Referring to FIG. 41, the button member 700 further moves downward to move the lock bar 610 downward. At this time, the lock bar 610 moves downward while elastically deforming the lock elastic member 620. Accordingly, the lock bar 610 releases the pushing on the second pushing portion 319. As the pushing of the second pushing portion 319 is released, the valve plate 310 is elastically restored, and the second channel valve 318 moves downward to open the channel P2. Thereafter, when the button member 700 moves upward, the lock bar 610 may be pushed upward by the elastic restoring force of the lock elastic member 620 to return to the initial state.

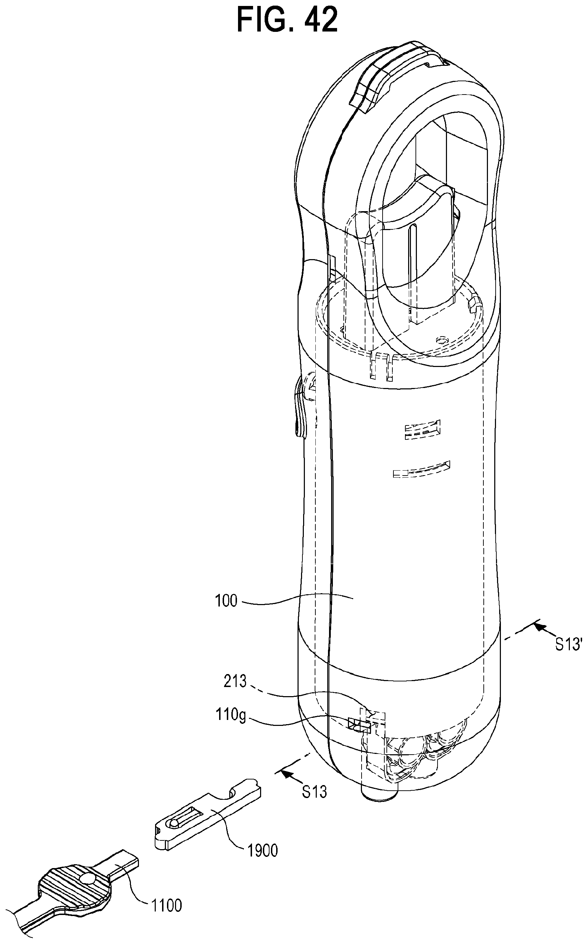

FIG. 42 is a perspective view illustrating a state in which the switching member 1900 is separated from the medicinal liquid control apparatus 80 of FIG. 3. FIGS. 43 and 44 are partial cross-sectional views of the medicinal liquid control apparatus 80 taken along line S13-S13' of FIG. 42, in which FIG. 43 illustrates a state in which the channel P1 is opened by the valve 316, and FIG. 44 illustrates a state in which the channel P1 is blocked by the valve 316 by the movement of the switching member 1900 from FIG. 43.

Hereinafter, a configuration related to the first channel valve 316 will be described with reference to FIGS. 42 to 44. By including the first channel valve 316, the medicinal liquid control apparatus 80 may allow only the medicinal liquid flowing through the second channel P2 to be injected into a patient by blocking the medicinal liquid continuously flowing along the first channel P1 as needed. In an embodiment, the medicinal liquid control apparatus 80 includes the switching member 1900 and the cotter 1100. The medicinal liquid control apparatus 80 may include the channel forming part 220 and the valve plate 310. The medicinal liquid control apparatus 80 may further include the valve support part 210.

The valve plate 310 may include the first pushing portion 317 that is pushed by the switching member 1900. The valve plate 310 may include the first channel valve 316 disposed and protruding on the side opposite to the first pushing portion 317. The first channel valve 316 may protrude upward toward the one end of the first extension hole 220h3 of the channel forming part 220. The valve plate 310 may further include the second channel valve 318.

The switching member 1900 may be configured to be movable to operate the first channel valve 316. The switching member 1900 may operate the first channel valve 316 independently from the second channel valve 318. The switching member 1900 may be configured to be movable in a direction transverse to the upper-lower direction on the valve support part 210. The switching member 1900 may be formed to extend in a direction transverse to the upper-lower direction. The switching member 1900 may be disposed inside the valve support part 210.

A groove 1920 into which the lower end of the first pushing portion 317 is insertable may be formed at the switching member 1900. The groove 1920 may be formed at the upper surface of the switching member 1900. In the released state in which the lower end of the first pushing portion 317 is inserted into the groove 1920 of the switching member 1900, the first channel valve 316 opens the one end of the first extension hole 220h3. In the locked state in which the lower end of the first pushing portion 317 is pressed upward by the upper surface of the switching member 1900, the first channel valve 316 blocks the one end of the first extension hole 220h3. In order to prevent the first pushing portion 317 from being engaged with a portion partitioning the groove 1920 when coming out from the groove 1920, the lower surface of the first pushing portion 317 may include an inclined surface directed upward in a direction opposite to the movement direction of the switching member 1900. The upper end of the portion partitioning the groove 1920 may be chamfered.

A switching member arrangement hole 213 into which the switching member 1900 is inserted is formed at the valve support part 210. The switching member arrangement hole 213 may be formed at a side surface of the valve support part. The movement path of the switching member 1900 is guided by the switching member arrangement hole 213. A portion of the cotter 1100 may be inserted into the switching member arrangement hole 213.

One of the valve support part 210 and the switching member 1900 includes a position setting protrusion protruding toward the other one, and a positioning groove 216 into which a position setting protrusion 1910 is insertable may be formed at the other one. In the locked state in which the lower end of the first pushing portion 317 is pressed upward by the upper surface of the switching member 1900, the position setting protrusion 1910 may be engaged with the positioning groove 216.

In the present embodiment, the switching member 1900 includes the position setting protrusion 1910, and the positioning groove 216 is formed at the valve support part 210. However, in another embodiment, the valve support part 210 may include the position setting protrusion and the positioning groove may be formed at the switching member 1900.

The position setting protrusion 1910 may protrude upward from the upper surface of the switching member 1900. The switching member 1900 may have a fixed end in one direction and a portion extending in the other direction to form a free end, and the position setting protrusion 1910 may be formed above the free end portion.

The switching hole 110g, 124 into which the cotter 1100c is insertable to push the switching member 1900 may be formed at the case 100. The cotter 1100 may be configured to be insertable into the case 100 to move the switching member 1900. The cotter 1100 may be inserted through the switching hole 110g, 124. The cotter 1100 may be configured to be insertable into the case 100 to lock the button member 700. The cotter 1100 may press the switching member 1900 in a direction transverse to the upper-lower direction, and accordingly, the switching member may move in a direction transverse to the upper-lower direction.

Hereinafter, the process of operating the switching member 1900, the cotter 1100, the first channel valve 316, and the like will be described with reference to FIGS. 43 and 44.

Referring to FIG. 43, in the initial state in which the switching member 1900 is not pushed in a preset direction (*see* M4 in FIG. 3), the first pushing portion 317 is inserted into the groove 1920 of the switching member 1900, and the first channel valve 316 opens the channel P1. At this time, the position setting protrusion 1910 of the switching member 1900 is in the state of being separated from the positioning groove 216 of the valve support part 210.

Referring to FIG. 44, by inserting the cotter 1100c into the switching hole 110g, 124 of the case 100, and pushing the switching member 1900 in the preset direction M4, the switching member 1900 is movable in the preset direction M4. Accordingly, the lower end of the first pushing portion 317 is separated from the groove 1920 of the switching member 1900. The upper surface of the switching member 1900 may push the first pushing portion 317 upward, the valve plate 310 may be elastically deformed, and the first channel valve 316 may move upward to block the first channel P1. At this time, the position setting protrusion 1910 of the switching member 1900 is inserted into the positioning groove 216 of the valve support part 210.

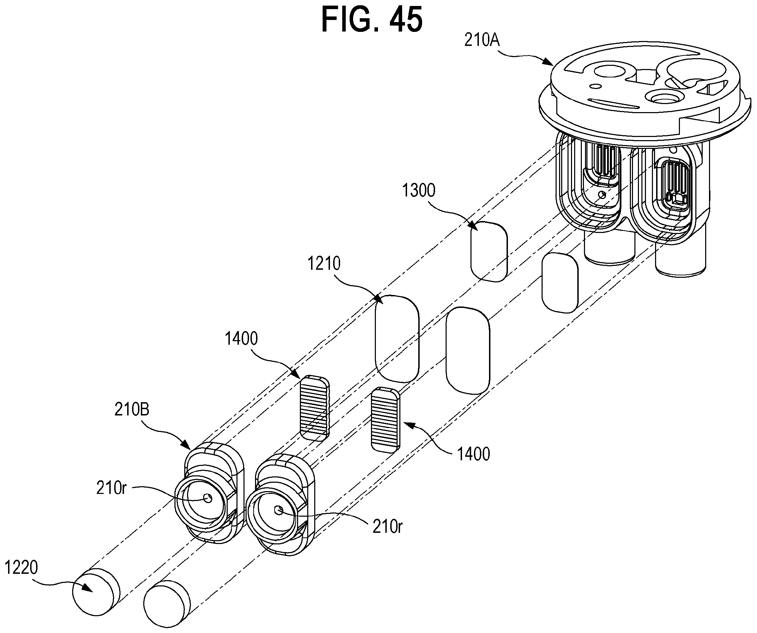

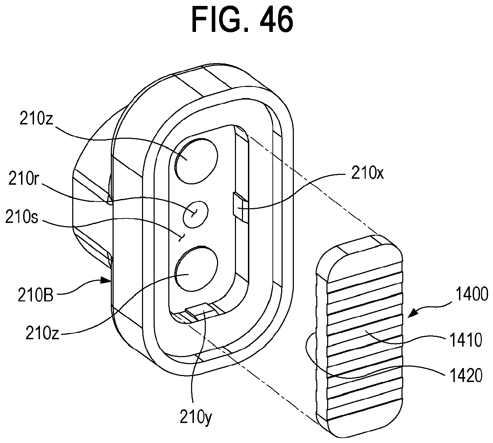

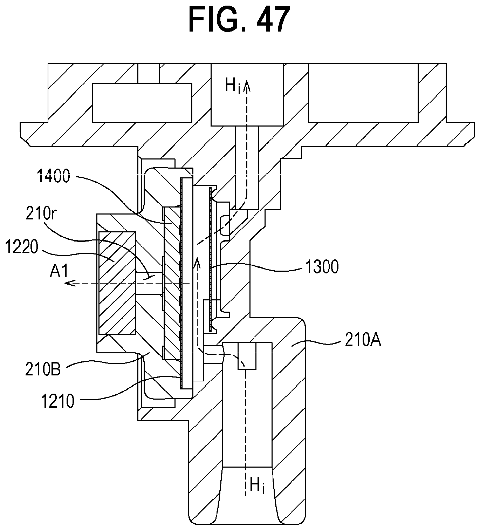

FIG. 45 is an exploded perspective view of the filter body 210A, the filter cap 210B, the first air passing filter 1210, the second air passing filter 1220, the boundary filter 1300, and the filter spacer 1400 of FIG. 6. FIG. 46 is an exploded perspective view illustrating a state in which the filter cap 210B and the filter spacer 1400 of FIG. 45 are separated from each other. FIG. 47 is a cross-sectional view of an assembly of components of FIG. 45 taken along line S12-S12' in FIG. 43.

Referring to FIGS. 45 to 47, in an embodiment, the medicinal liquid control apparatus 80 may include the air passing filter 1210 and the filter spacer 1400. The medicinal liquid control apparatus 80 may further include the filter cap 210B. The medicinal liquid control apparatus 80 may further include the second air passing filter 1220. The medicinal liquid control apparatus 80 may further include the boundary filter 1300.

The air passing filter 1210 is configured to discharge the air contained in the medicinal liquid to the outside. In the present embodiment, the air passing filter 1210 is formed at each of the inlet channel side and the outlet passage side, but only one of the inlet channel side and the outlet passage side may be provided with the air passing filter 1210. In addition, an air filter apparatus having a single channel (i.e., including only the first channel without the second channel) may be provided with the air passing filter 1210. The filter spacer 1400, the filter cap 210B, the filter body 210A, the second air passing filter 1220, and/or the boundary filter 1300 corresponding to the air passing filter 1210 may be provided.

An air passage branched from the medicinal liquid channel and connected to the outside may be formed at the medicinal liquid control apparatus 80. The air passing filter 1210 may be disposed at a boundary between the air passage and the medicinal liquid channel. The air passing filter may be a hydrophobic filter. The air passing filter 1210 may be disposed on the filter cap 210B. The filter cap 210B may provide the air passage.

The filter spacer 1400 separates the air passing filter 1210 from the inner surface that partitions the air passage of the filter cap 210B. The filter spacer 1400 is inserted into the filter cap 210B. The filter spacer 1400 is disposed on the rear surface side of the air passing filter 1210. The filter spacer 1400 may be disposed to be in contact with the air passing filter 1210. The filter spacer 1400 is configured to prevent or reduce a phenomenon in which the air passing filter 1210 is bent to the downstream side by the pressure of the medicinal liquid.

The surface of the filter spacer 1400 facing the air passing filter 1210 may be configured to provide protrusions 1410 to allow air to flow through the gaps between the protrusions 1410. For example, the protrusions 1410 may protrude in an embossed or wrinkled shape. The surface of the filter spacer 1400 opposite to the surface facing the air passing filter 1210 may form protrusions 1420 to allow air to flow through the gaps between the protrusions 1420. For example, the protrusions 1420 may protrude in an embossed or wrinkled shape.

An arrangement groove 210s into which the filter spacer 1400 is inserted may be formed at a filter cap 220B. The filter cap 220B may include a spacing portion 210x separating the side surface of the filter spacer 1400 from the inner surface of the filter cap 220B. The filter cap 220B may include a spacing portion 210y separating the other side surface of the filter spacer 1400 from the inner surface of the filter cap 220B. The filter cap 220B may include a spacing portion 210z separating the rear surface of the filter spacer 1400 from the inner surface of the filter cap 220B. By this, air may smoothly flow between the filter spacer 1400 and the inner surface of the filter cap 220B. An air hole 210r constituting a portion of the air passage may be formed at the filter cap 220B.

The technical idea of the present disclosure has been described heretofore with reference to some embodiments and examples illustrated in the accompanying drawings. However, it is to be understood that various substitutions, modifications and alterations may be made without departing from the technical idea and scope of the present disclosure that can be understood by those of ordinary skill in the technical field to which the present disclosure pertains. In addition, it is to be understood that such substitutions, modifications, and alterations fall within the appended claims.

## Claims

1. A medicinal liquid pushing apparatus having a medicinal liquid channel that guides a flow of a medicinal liquid, the medicinal liquid pushing apparatus comprising:
a reservoir defining an internal space configured to store the medicinal liquid on the medicinal liquid channel;
a reservoir pushing member configured to press the reservoir downward;
a button member disposed to be spaced apart upward from the reservoir pushing member and configured to be movable downward; and
a pressing member disposed between the button member and the reservoir pushing member and configured such that a lower end of the pressing member presses the reservoir pushing member downward while the pressing member is compressively and elastically deformed in an upper-lower direction when an upper end of the pressing member is pushed downward by the button member.

2. The medicinal liquid pushing apparatus of claim 1, wherein the reservoir pushing member is configured to move downward by being pushed downward by the pressing member without coming into contact with the button member.

3. The medicinal liquid pushing apparatus of claim 1, further comprising:
a support slider disposed in the button member and configured to move upward and downward together with the button member, the support slider including an engagement protrusion protruding in an engagement direction transverse to the upper-lower direction; and
a guide part that defines an engagement groove into which the engagement protrusion is insertable,
wherein the engagement protrusion is configured to be inserted into the engagement groove when the button member moves downward and reaches a predetermined position.

4. The medicinal liquid pushing apparatus of claim 3, wherein the button member and the support slider are configured to be engaged so as not to move upward in a state in which the engagement protrusion is inserted into the engagement groove.

5. The medicinal liquid pushing apparatus of claim 3, wherein the pressing member is configured to push the reservoir pushing member downward by being elastically restored while being stretched in the upper-lower direction in a state in which the engagement protrusion is inserted into the engagement groove.

6. The medicinal liquid pushing apparatus of claim 3, wherein the reservoir pushing member further includes a slide protrusion protruding upward and configured to be slidable in the upper-lower direction with respect to the support slider, and
wherein the support slider further includes:
a contact portion brought into contact with the slide protrusion in a release direction that is opposite to the engagement direction; and
a connection extension portion extending to connect the engagement protrusion and the contact portion to each other.

7. The medicinal liquid pushing apparatus of claim 6, wherein the slide protrusion includes:
a first portion constituting a lower portion of the slide protrusion and including a contact surface configured to come into contact with the contact portion; and
a second portion constituting an upper portion of the slide protrusion and including a contact surface configured to come into contact with the contact portion,
wherein the contact surface of the first portion is located in the engagement direction side with reference to the contact surface of the second portion.

8. The medicinal liquid pushing apparatus of claim 7, further comprising:
a slider spring configured to press the contact portion in the release direction.

9. The medicinal liquid pushing apparatus of claim 7, wherein the connection extension portion is configured to be compressively and elastically deformable in the engagement direction.

10. The medicinal liquid pushing apparatus of claim 6, wherein the support slider includes a plurality of engagement operation portions extending in engagement directions, respectively, around the slide protrusion, and
wherein each of the plurality of engagement operation portions includes the engagement protrusion, the contact portion, and the connection extension portion.

11. The medicinal liquid pushing apparatus of claim 10, wherein the support slider further includes an elastic connection portion that extends to interconnect any two adjacent engagement operation portions among the plurality of engagement operation portions and is configured to be elastically deformable.

12. The medicinal liquid pushing apparatus of claim 3, wherein the button member includes a lower part configured to press the upper end of the pressing member and disposed below the support slider.

13. The medicinal liquid pushing apparatus of claim 12, wherein the reservoir pushing member further includes a slide protrusion protruding upward and configured to be slidable in the upper-lower direction with respect to the support slider,
wherein the support slider further includes:
a contact portion brought into contact with the slide protrusion in a release direction that is opposite to the engagement direction; and
a connection extension portion extending to connect the engagement protrusion and the contact portion to each other, and
wherein a hole into which the slide protrusion is inserted is formed at the lower part.

14. The medicinal liquid pushing apparatus of claim 3, wherein the guide part extends along a circumference of the button member and is configured to guide a moving direction of the button member.

15. The medicinal liquid pushing apparatus of claim 1, further comprising:
a reservoir support part configured to support a lower surface of the reservoir.

16. The medicinal liquid pushing apparatus of claim 1, wherein the medicinal liquid channel includes:
a first channel that guides the medicinal liquid to flow from an inlet to an outlet; and
a second channel that guides the medicinal liquid to split at a branching point of the first channel, guides the split medicinal liquid to merge at a merging point located downstream of the branching point of the first channel, and is configured to be capable of opening/closing,
wherein the internal space of the reservoir is located on the second channel.

17. A medicinal liquid injection apparatus comprising:
the medicinal liquid pushing apparatus according to any one of claims 1 to 16;
a pumping module configured to press the medicinal liquid; and
an extension tube configured to allow the medicinal liquid flowing out from the pumping module by being pressed in the pumping module to flow through the extension tube,
wherein the medicinal liquid channel of the medicinal liquid pushing apparatus is connected to the extension tube.
